# EUROPEAN PATENT SPECIFICATION

(11) **EP 4 294 341 B1**
(45) Date of publication and mention of the grant of the patent: **22.07.2026**
(21) Application number: 22701428.9
(22) Date of filing: 20.01.2022
(51) Int. Cl.: A61F 13/05, A61M 1/00

(54) **DRESSING INTERFACE HAVING BUBBLE SUPPORTS WITH OPTIONAL BREAK-LINES**
VERBANDSCHNITTSTELLE MIT BLASENSTÜTZEN MIT OPTIONALEN BRUCHLINIEN
INTERFACE DE PANSEMENT AYANT DES SUPPORTS À BULLES AVEC DES LIGNES DE RUPTURE OPTIONNELLES

(30) Priority: 18.02.2021 US 202163150934 P
(43) Date of publication of application: 27.12.2023
(73) Proprietor: KCI Manufacturing Unlimited Company, Athlone, Co. Westmeath, N37XF22 (IE)
(72) Inventor: GONZALEZ, Javier, San Antonio, Texas 78265 (US)
(74) Representative: Simmons & Simmons
(86) International application number: PCT/IB2022/050470
(87) International publication number: WO 2022/175764

(56) References cited:
- WO-A1-2018/226669
- WO-A1-2019/177683
- WO-A1-2020/056182
- WO-A1-2020/217179
- WO-A1-2021/024165

## Description

### TECHNICAL FIELD

The invention set forth in the appended claims relates generally to tissue treatment systems and more particularly, but without limitation, to dressings and/or dressing interfaces for tissue treatment.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds . Regardless of the etiology of a wound, whether trauma, surgery, or another cause, proper care of the wound is important to the outcome . Treatment of wounds or other tissue with reduced pressure may be commonly referred to as "negative -pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.

Prior art is disclosed in WO2019/177683, WO2021/024165 and WO2020/056182.

### BRIEF SUMMARY

The invention is defined by the independent claim. A selection of optional features of the invention is set out in the dependent claims.

Insofar as the term invention or embodiment is used in the following, or features are presented as being optional, this should be interpreted in such a way that the only protection sought is that of the invention claimed (with due regard to Article 69 EPC and the protocol thereto).

References to "embodiment(s)" throughout the description which are not under the scope of the appended claims merely represent possible exemplary executions and are not part of the present invention.

### BRIEF DESCRIPTION OF THE DRAWINGS

Figure 1 is a block diagram of an example embodiment of a therapy system that can provide negative-pressure treatment and instillation treatment in accordance with this specification;
Figure 2 is a schematic, plan view of an illustrative embodiment of a tissue interface that may be associated with some embodiments of the therapy system of Figure 1 ,
Figure 3 is a top view of an example configuration of a bubble manifold that may be included in some embodiments of the tissue interface of Figure 2;
Figure 4 is a section view illustrating additional details that may be associated with some embodiments of the bubble manifold of Figure 3;
Figure 5 is a top view of an example configuration of a bubble manifold that may be included in some additional embodiments of the tissue interface of Figure 2;
Figure 6 is a section view illustrating additional details that may be associated with some embodiments of the bubble manifold of Figure 5;
Figure 7 is a section view illustrating details that may be associated with some additional embodiments of the bubble manifold of Figure 5;
Figure 8 is a section view illustrating details that may be associated with some additional embodiments of the bubble manifold of Figure 5;
Figure 9 is a top view of another example configuration of a bubble manifold that may be included in some additional embodiments of the tissue interface of Figure 2;
Figure 10 is a top view of another example configuration of a bubble manifold that may be associated with some additional embodiments of the tissue interface of Figure 2;
Figure 11 is a section view illustrating additional details that may be associated with some embodiments of the bubble manifold of Figure 10;
Figure 12 is a section view illustrating details that may be associated with some additional embodiments of the bubble manifold of Figure 10;
Figure 13 is a section view illustrating details that may be associated with some additional embodiments of the bubble manifold of Figure 10;
Figure 14 is a top view of another example configuration of a bubble manifold that may be associated with some additional embodiments of the tissue interface of Figure 2;
Figure 15 is a top view of another example configuration of a bubble manifold that may be associated with some additional embodiments of the tissue interface of Figure 2;
Figure 16 is a schematic, plan view of another illustrative embodiment of a tissue interface that may be associated with some additional embodiments of the therapy system of Figure 1;
Figure 17 is a section view illustrating additional details that may be associated with some embodiments of the tissue interface of Figure 16;
Figure 18 is a section view illustrating additional details that may be associated with some embodiments of the tissue interface of Figure 16;
Figure 19 is a schematic, plan view of another illustrative embodiment of a tissue interface that may be associated with some further embodiments of the therapy system of Figure 1;
Figure 20 is a section view illustrating additional details that may be associated with an example embodiment of the tissue interface of Figure 19;
Figure 21 is a section view illustrating additional details that may be associated with another example embodiment of the tissue interface of Figure 19;
Figure 22 is a schematic, cut-away plan view of another illustrative embodiment of a tissue interface that may be associated with some additional embodiments of the therapy system of Figure 1;
Figure 23 is a section view illustrating additional details that may be associated with some example embodiments of the tissue interface of Figure 22;
Figure 24A is a section view of another illustrative embodiment of a tissue interface that may be associated with some embodiments of the therapy system of Figure 1;
Figure 24B is a section view of another illustrative embodiment of a tissue interface that may be associated with some embodiments of the therapy system of Figure 1;
Figure 25 is a schematic diagram, with a portion in cross-section, of an illustrative dressing for treating a tissue site, which may be associated with some embodiments of the therapy system of Figure 1;
Figure 26 is a schematic diagram illustrating details that may be associated with an example method of operating the therapy system of Figure 1;
Figure 27 is a schematic, plan view of an illustrative embodiment of another tissue interface that may be associated with some embodiments of the therapy system of Figure 1, illustrating additional details that may be associated with some embodiments;
Figure 28 is an isometric view of the embodiment of Figure 27;
Figure 29 is a section view of Figure 27, illustrating additional details that may be associated with some embodiments;
Figure 30 is a section view of another example embodiment of Figure 28, illustrating additional details that may be associated with some embodiments;
Figure 31 is an isometric view of an illustrative embodiment of yet another tissue interface that may be associated with some embodiments of the therapy system of Figure 1, illustrating additional details that may be associated with some embodiments;
Figure 32 is a section view of an example cross-section of Figure 31, illustrating additional details that may be associated with some embodiments;
Figure 33 is a section view of another example cross-section of Figure 31, illustrating additional details that may be associated with some embodiments;
Figure 34 is a section view of yet another example cross-section of Figure 31, illustrating additional details that may be associated with some embodiments;
Figure 35 is a schematic diagram, with a portion in cross-section, of an illustrative dressing for treating a tissue site, which may comprise a tissue interface similar to that of Figure 31 and may be associated with some embodiments of the therapy system of Figure 1;
Figure 36 is a schematic, plan view of an illustrative embodiment of still another tissue interface that may be associated with some embodiments of the therapy system of Figure 1, illustrating additional details that may be associated with some embodiments;
Figure 37 is a section view of an example cross-section of Figure 36, illustrating additional details that may be associated with some embodiments;
Figure 38 is a section view of another example cross-section of Figure 36, illustrating additional details that may be associated with some embodiments;
Figure 39 is a schematic, plan view of an illustrative embodiment of still another tissue interface that may be associated with some embodiments of the therapy system of Figure 1, illustrating additional details that may be associated with some embodiments;
Figure 40 is a section view of an example cross-section of Figure 39, illustrating additional details that may be associated with some embodiments;
Figure 41 is a section view of another example cross-section of Figure 39, illustrating additional details that may be associated with some embodiments;
Figure 42 is a schematic, plan view of an illustrative embodiment of still another tissue interface that may be associated with some embodiments of the therapy system of Figure 1, illustrating additional details that may be associated with some embodiments;
Figure 43 is a schematic, plan view of an illustrative embodiment of still another tissue interface that may be associated with some embodiments of the therapy system of Figure 1, illustrating additional details that may be associated with some embodiments;
Figure 44 is an isometric view of an illustrative embodiment of yet another tissue interface that may be associated with some embodiments of the therapy system of Figure 1, illustrating additional details that may be associated with some embodiments;
Figure 45 is a schematic, plan view of an illustrative embodiment of the tissue interface of Figure 44, illustrating additional details that may be associated with some embodiments;
Figure 46 is a section view of an example cross-section of Figure 45, illustrating additional details that may be associated with some embodiments;
Figure 47 is a section view of another example cross-section of Figure 45, illustrating additional details that may be associated with some embodiments;
Figure 48 is a section view of an alternate example cross-section of a tissue interface, illustrating additional details that may be associated with some embodiments;
Figure 49 is a section view of another alternate example cross-section of a tissue interface, illustrating additional details that may be associated with some embodiments; and
Figure 50 is a schematic diagram, with a portion in cross-section, of an illustrative dressing for treating a tissue site, which may comprise a tissue interface similar to that of Figure 48 or Figure 49 and may be associated with some embodiments of the therapy system of Figure 1.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

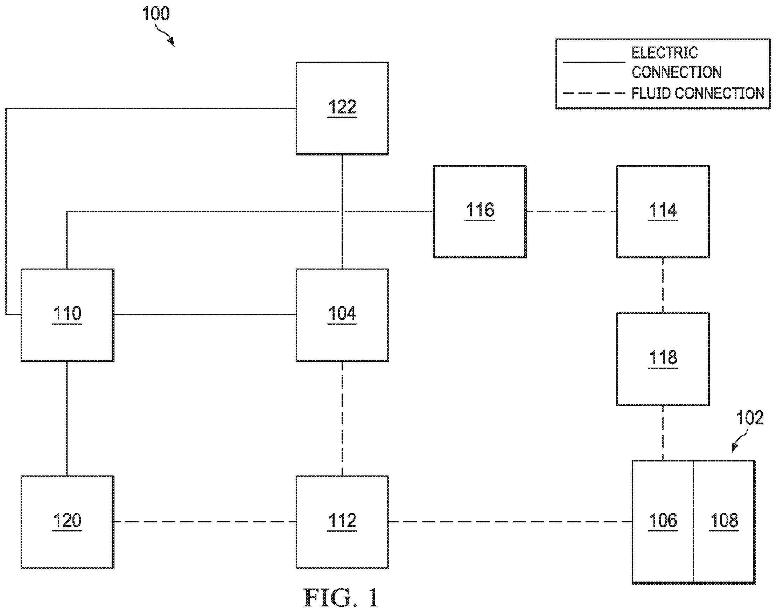

Figure 1 is a block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 104, and one or more distribution components. A distribution component is preferably detachable and may be disposable, reusable, or recyclable. A dressing, such as a dressing 102, and a fluid container, such as a container 112, are examples of distribution components that may be associated with some examples of the therapy system 100. As illustrated in the example of Figure 1, the dressing 102 may comprise or consist essentially of a tissue interface 108, a cover 106, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 102. For example, such a dressing interface may be a SENSAT.R.A.C.^{™} Pad available from Kinetic Concepts, Inc. of San Antonio, Texas.

The therapy system 100 may also include a regulator or controller, such as a controller 110. Additionally, the therapy system 100 may include sensors to measure operating parameters and provide feedback signals to the controller 110 indicative of the operating parameters. As illustrated in Figure 1, for example, the therapy system 100 may include a first sensor 120 and a second sensor 122 coupled to the controller 110.

The therapy system 100 may also include a source of instillation solution. For example, a solution source 114 may be fluidly coupled to the dressing 102, as illustrated in the example embodiment of Figure 1. The solution source 114 may be fluidly coupled to a positive-pressure source such as a positive-pressure source 116, a negative-pressure source such as the negative-pressure source 104, or both in some embodiments. A regulator, such as an instillation regulator 118, may also be fluidly coupled to the solution source 114 and the dressing 102 to ensure proper dosage of instillation solution (e.g. saline) to a tissue site. For example, the instillation regulator 118 may comprise a piston that can be pneumatically actuated by the negative-pressure source 104 to draw instillation solution from the solution source during a negative-pressure interval and to instill the solution to a dressing during a venting interval. Additionally or alternatively, the controller 110 may be coupled to the negative-pressure source 104, the positive-pressure source 116, or both, to control dosage of instillation solution to a tissue site. In some embodiments, the instillation regulator 118 may also be fluidly coupled to the negative-pressure source 104 through the dressing 102, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 104 may be combined with the controller 110, the solution source 114, and other components into a therapy unit.

In general, components of the therapy system 100 may be coupled directly or indirectly. For example, the negative-pressure source 104 may be directly coupled to the container 112 and may be indirectly coupled to the dressing 102 through the container 112. Coupling may include fluid, mechanical, thermal, electrical, or chemical coupling (such as a chemical bond), or some combination of coupling in some contexts. For example, the negative-pressure source 104 may be electrically coupled to the controller 110 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 104, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 104 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 112 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 110, may be a microprocessor or computer programmed to operate one or more components of the therapy system 100, such as the negative-pressure source 104. In some embodiments, for example, the controller 110 may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the tissue interface 108, for example. The controller 110 is also preferably configured to receive one or more input signals, such as a feedback signal, and programmed to modify one or more operating parameters based on the input signals.

Sensors, such as the first sensor 120 and the second sensor 122, are generally known in the art as any apparatus operable to detect or measure a physical phenomenon or property, and generally provide a signal indicative of the phenomenon or property that is detected or measured. For example, the first sensor 120 and the second sensor 122 may be configured to measure one or more operating parameters of the therapy system 100. In some embodiments, the first sensor 120 may be a transducer configured to measure pressure in a pneumatic pathway and convert the measurement to a signal indicative of the pressure measured. In some embodiments, for example, the first sensor 120 may be a piezo-resistive strain gauge. The second sensor 122 may optionally measure operating parameters of the negative-pressure source 104, such as a voltage or current, in some embodiments. Preferably, the signals from the first sensor 120 and the second sensor 122 are suitable as an input signal to the controller 110, but some signal conditioning may be appropriate in some embodiments For example, the signal may need to be filtered or amplified before it can be processed by the controller 110. Typically, the signal is an electrical signal, but may be represented in other forms, such as an optical signal.

The tissue interface 108 can be generally adapted to partially or fully contact a tissue site. The tissue interface 108 may take many forms, and may have many sizes, shapes, or thicknesses, depending on a variety of factors, such as the type of treatment being implemented or the nature and size of a tissue site. For example, the size and shape of the tissue interface 108 may be adapted to the contours of deep and irregular shaped tissue sites. Any or all of the surfaces of the tissue interface 108 may have an uneven, coarse, or jagged profile.

In some embodiments, the tissue interface 108 may comprise or consist essentially of a manifold. A manifold in this context may comprise or consist essentially of a means for collecting or distributing fluid across the tissue interface 108 under pressure. For example, a manifold may be adapted to receive negative pressure from a source and distribute negative pressure through multiple apertures across the tissue interface 108, which may have the effect of collecting fluid from across a tissue site and drawing the fluid toward the source. In some embodiments, the fluid path may be reversed or a secondary fluid path may be provided to facilitate delivering fluid, such as fluid from a source of instillation solution, across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections or supports that form interconnected fluid pathways. For example, a manifold may be molded to provide surface projections, such as bubbles, that define interconnected fluid pathways.

In some embodiments, a manifold may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of a manifold may also vary according to needs of a prescribed therapy. For example, the tensile strength of foam may be increased for instillation of topical treatment solutions. The 25% compression load deflection of the manifold may be at least 0.35 pounds per square inch, and the 65% compression load deflection may be at least 0.43 pounds per square inch. In some embodiments, the tensile strength of the manifold may be at least 10 pounds per square inch. The manifold may have a tear strength of at least 2.5 pounds per inch. In some embodiments, the manifold may be foam comprised of polyols such as polyester or polyether, isocyanate such as toluene diisocyanate, and polymerization modifiers such as amines and tin compounds. In some examples, the manifold may be reticulated polyurethane foam such as found in GRANUFOAM^{™} Dressing or V.A.C. VERAFLO^{™} Dressing, both available from Kinetic Concepts, Inc. of San Antonio, Texas.

A manifold may be either hydrophobic or hydrophilic. In an example in which a manifold is hydrophilic, the manifold may also wick fluid away from a tissue site, while continuing to distribute negative pressure to the tissue site. The wicking properties of the manifold may draw fluid away from a tissue site by capillary flow or other wicking mechanisms. An example of a hydrophilic material that may be suitable is a polyvinyl alcohol, open-cell foam such as V.A.C. WHITEFOAM^{™} Dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, a manifold may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. A manifold may further serve as a scaffold for new cell-growth, or a scaffold material may be used in conjunction with the manifold to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may comprise or consist of, for example, an elastomeric film or membrane that can provide a seal adequate to maintain a negative pressure at a tissue site for a given negative-pressure source. The cover 106 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 106 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 106 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 106 may comprise INSPIRE 2301 having an MVTR (upright cup technique) of 2600 g/m²/24 hours and a thickness of about 30 microns.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive configured to bond the cover 106 to epidermis around a tissue site. In some embodiments, for example, some or all of the cover 106 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 114 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions

In operation, the tissue interface 108 may be placed within, over, on, or otherwise proximate to a tissue site. If the tissue site is a wound, for example, the tissue interface 108 may partially or completely fill the wound, or it may be placed over the wound. The cover 106 may be placed over the tissue interface 108 and sealed to an attachment surface near a tissue site. For example, the cover 106 may be sealed to undamaged epidermis peripheral to a tissue site. Thus, the dressing 102 can provide a sealed therapeutic environment proximate to a tissue site, substantially isolated from the external environment, and the negative-pressure source 104 can reduce pressure in the sealed therapeutic environment.

The process of reducing pressure may be described illustratively herein as "delivering," "distributing," or "generating" negative pressure, for example.

In general, exudate and other fluid flow toward lower pressure along a fluid path. Thus, the term "downstream" typically refers to a location in a fluid path relatively closer to a source of negative pressure or further away from a source of positive pressure. Conversely, the term "upstream" may refer to a location relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source.

Negative pressure applied across the tissue site through the tissue interface 108 in the sealed therapeutic environment can induce macro-strain and micro-strain in the tissue site. Negative pressure can also remove exudate and other fluid from a tissue site, which can be collected in container 112.

In some embodiments, the controller 110 may receive and process data from one or more sensors, such as the first sensor 120. The controller 110 may also control the operation of one or more components of the therapy system 100 to manage the pressure delivered to the tissue interface 108. In some embodiments, controller 110 may include an input for receiving a desired target pressure and may be programmed for processing data relating to the setting and inputting of the target pressure to be applied to the tissue interface 108. In some example embodiments, the target pressure may be a fixed pressure value set by an operator as the target negative pressure desired for therapy at a tissue site and then provided as input to the controller 110. The target pressure may vary from tissue site to tissue site based on the type of tissue forming a tissue site, the type of injury or wound (if any), the medical condition of the patient, and the preference of the attending physician. After selecting a desired target pressure, the controller 110 can operate the negative-pressure source 104 in one or more control modes based on the target pressure and may receive feedback from one or more sensors to maintain the target pressure at the tissue interface 108.

The controller 110 may function according to one or more control modes. In some embodiments, the controller 110 may have a continuous pressure mode, in which the negative-pressure source 104 is operated to provide a constant target negative pressure for the duration of treatment or until manually deactivated. Additionally or alternatively, the controller 110 may have an intermittent pressure mode. In some examples, the controller 110 can operate the negative-pressure source 104 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 104, which can operate according to a square wave pattern between the target pressure and atmospheric pressure.

In some example embodiments, the increase in negative-pressure from ambient pressure to the target pressure may not be instantaneous. For example, the negative-pressure source 104 and the dressing 102 may have an initial rise time. The initial rise time may vary depending on the type of dressing and therapy equipment being used. For example, the initial rise time for one therapy system may be in a range of about 20-30 mmHg/second and in a range of about 5-10 mmHg/second for another therapy system. If the therapy system 100 is operating in an intermittent mode, the repeating rise time may be a value substantially equal to the initial rise time.

According to another example pressure control mode, such as a dynamic pressure mode, the target pressure can vary with time. For example, the target pressure may vary in the form of a triangular waveform, varying between a negative pressure of 50 and 135 mmHg with a rise time set at a rate of +25 mmHg/min. and a descent time set at -25 mmHg/min. In other embodiments of the therapy system 100, the triangular waveform may vary between negative pressure of 25 and 135 mmHg with a rise time set at a rate of +30 mmHg/min and a descent time set at -30 mmHg/min.

In some embodiments, the controller 110 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 110, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

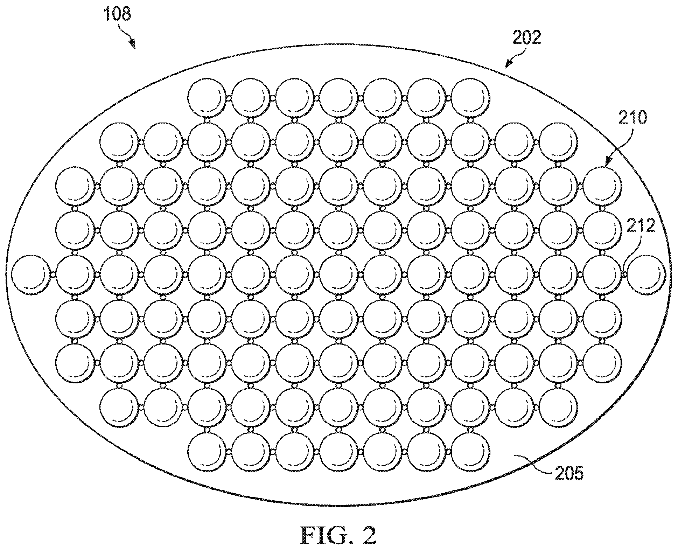

Figure 2 is a schematic top-view of an example of the tissue interface 108, illustrating additional details that may be associated with some embodiments. The tissue interface 108 generally comprises or consists essentially of a manifold or a manifold layer. The tissue interface 108 may be adapted to provide negative pressure from the negative-pressure source 104 of the therapy system 100 to a tissue site, and to collect and transport fluid extracted from the tissue site. For example, the tissue interface 108 may be adapted to receive negative pressure from the negative-pressure source 104 and distribute the negative pressure through multiple apertures across the tissue interface 108, which may have the effect of collecting fluid from across a tissue site and drawing the fluid toward the negative-pressure source 104. In some embodiments, the fluid path may be reversed or a secondary fluid path may be provided to facilitate delivering fluid, such as from a source of instillation solution, across the tissue interface 108. The tissue interface 108 may comprise a bubble manifold 202, which may include a plurality of bubbles 210 extending from at least one liquid-impermeable layer 205. The bubbles 210 may be in the form of and may be referred to as open-celled blisters, spacers, supports, protrusions, or other raised formations. In some additional embodiments, the bubbles 210 may be in the form of closed cells. Each of the plurality of bubbles 210 may comprise a substantially circular, oval, triangular, square, rectangular, or other shape, as appropriate. In some instances, triangular-shaped bubbles may maintain their height longer under compression, such as under the application of negative pressure within an abdominal space. Furthermore, the bubbles 210 may be textured, or include surface texture features. The bubble manifold 202 of the tissue interface 108 may additionally comprise apertures 212 positioned between the bubbles 210 to allow fluid transfer through the bubble manifold 202. In some embodiments, the apertures 212 may comprise perforations or fenestrations.

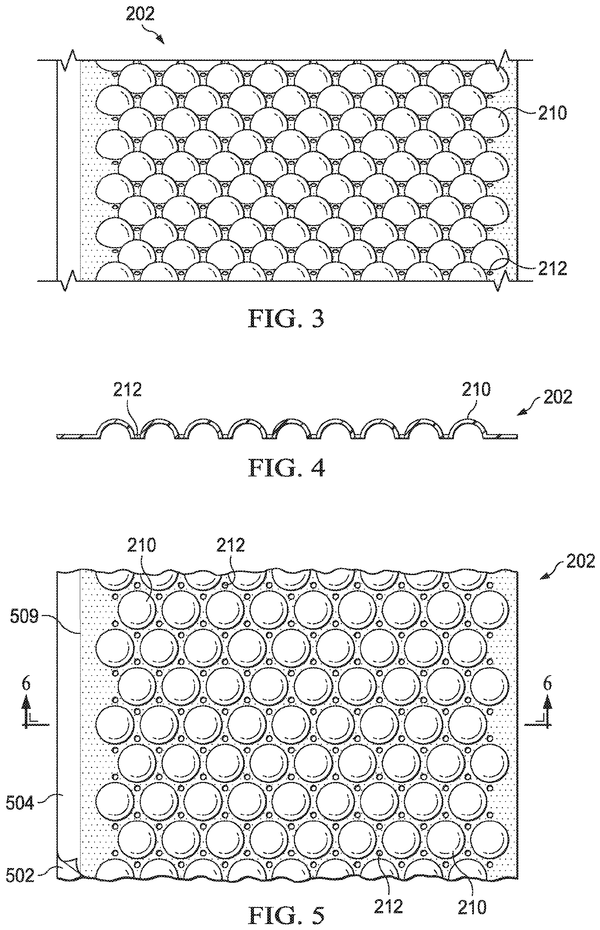

Figure 3 is a perspective view of an example of the bubble manifold 202 of Figure 2, illustrating additional details that may be associated with some embodiments. As illustrated in the example of Figure 3, the bubbles 210 may be in the form of blisters, and may be generally hemispherical and uniformly distributed in some embodiments. For example, the bubble manifold 202 may comprise or consist essentially of a film of liquid-impermeable material, such as a polyurethane material, having bubbles 210 in the form of blisters. In some embodiments, the blisters may comprise a plurality of raised formations that extend above or below a plane of the bubble manifold 202 (e.g. the liquid-impermeable layer 205). Within each of the blisters may be an empty cavity which may be open to the surrounding environment. For example, portions of a film of liquid-impermeable material that forms the bubble manifold 202 may be shaped or formed into the blisters. In some embodiments, the blisters may be in the form of small vacuum-formed regions of the film of the bubble manifold 202.

The bubbles 210 may have dimensions that depend on the particular application of the bubble manifold 202 and tissue interface 108. For example, the bubbles 210 may be in the form of blisters having a height of approximately 1.0 mm - 10.0 mm, approximately 1.0 mm - 6.0 mm, approximately 1.0 mm - 4.0 mm, approximately 3.0 mm - 6.0 mm, at least 3.0 mm, approximately 9.0 mm - 10.0 mm, at least 9.0 mm, and/or less than 10.0 mm; and may have a diameter or width of approximately 1.0 mm - 12.0 mm, approximately 1.0 mm - 5.0 mm, approximately 1.0 mm - 4.0 mm, approximately 3.0 mm- 5.0 mm, approximately 6.0 mm - 12.0 mm, approximately 9.0 mm - 12.0 mm, approximately 10.0 mm - 12.0 mm, approximately 12.0 mm, or approximately equal to the height. In some embodiments, the blisters may measure approximately 1.5 mm in height and approximately 1.5 mm in diameter. In some embodiments, the blisters may measure at least 3.0 mm in height and at least 3.0 mm in diameter. In some embodiments, the blisters may measure approximately 3.0 mm in height and/or approximately 3.0 mm - 5.0 mm in diameter or width. In some embodiments, the blisters may each measure approximately 9.0 mm - 10.0 mm in height and/or approximately 6.0 mm - 12.0 mm in diameter or width. In some embodiments, the distance or spacing between each of the blisters may be approximately 1.0 mm - 3.0 mm, and in some embodiments may have a spacing of approximately 2.0 mm or approximately 1.5 mm. In some embodiments, the distance or spacing between each of the blisters may be approximately 3.0 - 6.0 mm. In some embodiments, the distance or spacing between each of the blisters may be approximately half of the blister diameter or width. In some embodiments, each individual blister may be dome-shaped or hemispherically-shaped (e.g. half-spheres). Additionally or alternatively, the blisters may be in the form of raised formations having different shapes, such as generally conical, cylindrical, spherical, ovoidal, tubular having a flattened or hemispherical end, pyramidal, rectangular prism, cuboidal, or geodesic. Furthermore, the bubbles 210 may vary in size or spacing across a surface area of the bubble manifold 202. For example, the bubbles 210 may be smaller or larger in a central portion of the bubble manifold 202, and/or may gradually decrease or increase in size along a distance from a central portion to perimeter portion of the bubble manifold 202. For example, the bubbles 210 may be larger towards a perimeter portion of the bubble manifold 202, which may have the effect of mitigating potential pressure drops across the bubble manifold 202. Additionally, the bubbles 210 may be increasingly spaced further apart or more closely along a distance from a central portion to a perimeter portion of the bubble manifold 202. The shape of the bubbles 210 may also change along a distance from a central portion to a perimeter portion of the bubble manifold 202. Such variations in size, spacing/density, and shape may aid with deploying the bubble manifold 202 and tissue interface 108 to establish an appropriate fluid removal gradient at a tissue site.

The thickness of the bubble manifold 202 may also vary according to the needs of a prescribed therapy. For example, the thickness of the bubble manifold 202 may be decreased to relieve stress or tension on tissue at a tissue site. The thickness of the bubble manifold 202 can also affect the conformability of the tissue interface 108. In some embodiments, the bubble manifold 202 may comprise a film having a material thickness in a range of about 20 to 500 micrometers, or in some more specific embodiments in a range of about 50 to 150 micrometers. Depending on the particular embodiment, the orientation of the bubble manifold 202 may be reversed so that the bubbles 210 of the bubble manifold 202 may either face or extend upwards or downwards from a plane of the bubble manifold 202.

The bubble manifold 202 may additionally include apertures 212 positioned between the bubbles 210 to allow fluid transfer through the film of the bubble manifold 202. The number of apertures 212 may vary depending on the type of negative pressure and/or instillation therapy to be provided by the therapy system 100. The apertures 212 may have different shapes and sizes, and the apertures 212 may have a diameter, major axis, or length between about 0.5 mm and 1.5 mm. The apertures 212 may be fenestrations, in some embodiments. In some embodiments, the bubble manifold 202 may comprise a polyurethane film with vacuum-formed blisters that is subsequently fenestrated with slits. Additionally, the bubble manifold 202 may be formed with rings, which may be in the form of concentric circles, in regions of the bubble manifold 202 between areas of the bubble manifold 202 comprising bubbles 210. The rings may provide a visual cue to a user to aid with sizing of the tissue interface 108, which may include cutting. In some embodiments, the rings of the bubble manifold 202 may be formed during a vacuum-forming process of the bubble manifold 202, and may include weakened regions or designated areas of the bubble manifold 202 for cutting or otherwise sizing. For example, if the vacuum draw corresponding to the regions of the bubble manifold 202 where the rings are desired is higher than the vacuum applied to surrounding areas of the bubble manifold 202, weaknesses would be formed in desired areas that would allow for preferential tearing or cutting.

Figure 4 is a section view of an example embodiment of the bubble manifold 202 of Figure 3, illustrating additional details that may be associated with some embodiments. For example, the bubble manifold 202 may be formed of a single sheet or film of liquid-impermeable material, which may have the bubbles 210 and apertures 212 formed thereon. In some embodiments, the bubbles 210 may be in the form of blisters and may be formed in the bubble manifold 202 by applying a vacuum to the film of liquid-impermeable material of the bubble manifold 202 to create the blisters.

As shown in Figure 4, the apertures 212 may be formed in the portions of the bubble manifold 202 that are between the bubbles 210 and may extend through the film of liquid-impermeable material to permit fluids to flow through the bubble manifold 202. The number of apertures 212 may vary depending on the type of negative pressure and instillation therapy to be provided by the therapy system 100. The apertures 212 may have different shapes, such as, for example, circular, elliptical, rectangular, or other irregular shape. Such apertures 212 may have a diameter, major axis, or length between about 0.5 mm and 1.5 mm. In some example embodiments, the apertures 212 may be formed by cutting or perforating the liquid-impermeable material of the bubble manifold 202.

Figure 5 is a perspective view of a portion of another example of the bubble manifold 202 of Figure 2, illustrating additional details that may be associated with some embodiments. In some embodiments, the bubble manifold 202 may comprise a manifold and may be formed with a plurality of liquid-impermeable layers, e.g., a first layer 502 and a second layer 504. "Liquid impermeable" with respect to "liquid-impermeable layers" means that the layers are formed with a liquid-impermeable material. Thus, although formed with a liquid-impermeable material, the first layer 502 and the second layer 504 may be liquid permeable when fenestrated, perforated, or otherwise include fluid passageways, but nonetheless are referred to as liquid-impermeable layers. In some embodiments, the first layer 502 and the second layer 504 may be sealingly coupled or attached to one another in any suitable manner, such as, without limitation, by welding (e.g. heat welding, ultrasonic welding, RF welding, etc.), bonding, adhesives, cements, or other bonding devices. In some embodiments, the first layer 502 and the second layer 504 may comprise a non-adherent material, such as a medical drape, capable of inhibiting tissue from adhering to the medical drape. For example, in some embodiments, the first layer 502 and the second layer 504 may comprise a liquid-impermeable polymeric film, such as a breathable polyurethane film.

In some embodiments, each of the first layer 502 and the second layer 504 may comprise or consist essentially of a liquid-impermeable polymer film, having inner surfaces coupled to each other to form a sealed region 509 defining a plurality of bubbles 210, which may be in the form of closed cells. The inner surfaces of the first layer 502 and the second layer 504 may be coupled to each other to form bubbles 210 that are in the form of closed cells that are substantially airtight to inhibit excessive collapsing of the bubbles 210 from the application of negative pressure, which could block the flow of fluid through or along the bubble manifold 202.

The two sheets of liquid-impermeable, polymeric film, first layer 502 and second layer 504, may be in the form of a single sheet of material having two laminae or two separate sheets that are coupled together to form the bubbles 210. The sheets of liquid-impermeable, polymeric film may initially be separate sheets that are brought into superposition and sealed or they may be formed by folding a single sheet unto itself with a heat sealable surface facing inward. Each sheet of the liquid-impermeable polymeric film also may be a monolayer or multilayer structure depending on the application of the desired structure of the bubbles 210.

The sheets of liquid-impermeable, polymeric film may comprise any flexible material that can be manipulated to enclose the bubbles 210 formed of closed cells. For example, the bubble manifold 202 may be formed of two welded layers of polyolefin film that encapsulates air in pockets. Additionally or alternatively, various thermoplastic materials may be used for producing the film layers of the bubble manifold 202. Non-limiting examples of suitable thermoplastic polymers include polyethylene homopolymers, such as low density polyethylene (LDPE) and high density polyethylene (HDPE), and polyethylene copolymers, such as, ionomers, EVA, EMA, heterogeneous (Zeigler-Natta catalyzed) ethylene/alpha-olefin copolymers, and homogeneous (metallocene, single-cite catalyzed) ethylene/alpha-olefin copolymers. Ethylene/alpha-olefin copolymers are copolymers of ethylene with one or more comonomers selected from C₃ to C₂₀ alpha-olefins, such as 1-butene, 1-pentene, 1-hexene, 1-octene, and methyl pentene, in which the polymer molecules comprise long chains with relatively few side chain branches, including linear low-density polyethylene (LLDPE), linear medium-density polyethylene (LMDPE), very low-density polyethylene (VLDPE), and ultra-low-density polyethylene (ULDPE). Various other materials are also suitable such as, polypropylene homopolymer or polypropylene copolymer (e.g., propylene/ethylene copolymer), polyesters, polystyrenes, polyamides, polycarbonates, etc.

The bubbles 210 formed of closed cells may be preferably resistant to collapsing under therapeutic levels of negative pressure. In some embodiments, the bubbles 210 may be formed by a material having sufficient tensile strength to resist stretching under apposition forces of negative pressure. The tensile strength of a material is the ability of material to resist stretching as represented by a stress-strain curve, where stress is the force per unit area, i.e., pascals (Pa), newtons per square meter (N/m²), or pounds per square inch (psi). The ultimate tensile strength (UTS) is the maximum stress the material can withstand while being stretched before failing or breaking. Many materials display a linear elastic behavior defined by a linear stress-strain relationship often extending up to a nonlinear region represented by the yield point, i.e., the yield strength of a material. For example, high-density polyethylene (HDPE) has a high tensile strength and low-density polyethylene (LDPE) has a slightly lower tensile strength, both of which are suitable materials for forming the bubbles 210. Linear low-density polyethylene (LLDPE) may be used as well because the material stretches very little as the force is increased up to the yield point of the material. The yield strength of HDPE ranges from 26-33 MPa, and has a UTS of 37 MPa, while LDPE has somewhat lower values. In some example embodiments, the bubbles 210 may be formed from a material that has a yield strength greater than about 20 MPa.

In some example embodiments, the sealed region 509 may be formed by a heat seal (e.g. heat weld) between the inner surfaces of the first layer 502 and the second layer 504. Additionally or alternatively, the sealed region 509 may be formed by adhesion between the first layer 502 and the second layer 504. The first layer 502 and the second layer 504 may also be adhesively bonded to each other. The bubbles 210 may be substantially airtight closed cells when formed and have an internal pressure that is substantially an ambient pressure. In other embodiments, the bubbles 210 may be closed cells that are inflated with air or other suitable gas, such as, for example, carbon dioxide or nitrogen. The bubbles 210 may be closed cells that are inflated to have an internal pressure greater than the atmospheric pressure to maintain their shape and resistance to collapsing under pressure. For example, the bubbles 210 may be inflated to a pressure up to about 25 psi above the atmospheric pressure so that they do not collapse.

The sealed region 509 comprises sealed segments between the bubbles 210 that may be flexible enough so that the bubble manifold 202 is sufficiently flexible to conform to the shape of the tissue site. The sealed segments may be sufficiently flexible or sized so that the bubble manifold 202 may be folded into two or more layers. The sealed segments of the sealed region 509 may serve as common boundaries between adjacent bubbles 210. The sealed segments of the sealed region 509 may also be perforated to provide pathways for fluid to flow through the bubble manifold 202. In some example embodiments, the sealed region 509 may include a plurality of apertures 212 between the bubbles 210 in the sealed region 509 and extending through both the first layer 502 and the second layer 504 to permit fluid to flow through the bubble manifold 202. The number of apertures 212 may vary depending on the type of negative pressure and instillation therapy to be provided by the therapy system 100. The apertures 212 may have different shapes, such as, for example, circular, elliptical, rectangular, or other irregular shape. Such apertures 212 may have a diameter, major axis, or length between about 0.5 mm and 1.5 mm. In other example embodiments, the apertures 212 may be formed by perforating or cutting the segments of the sealed region 509.

As illustrated in the example of Figure 5, the sealed region 509 may define the base or the cross-sectional shape of each of the bubbles 210 as generally circular. Additionally or alternatively, the base of one or more of the bubbles 210 may have other shapes, such as rectangular, triangular, or hexagonal. The bubbles 210 may be formed with a three-dimensional shape corresponding to the cross-sectional shape of the bubbles 210. For example, the volumetric shape may be generally hemispherical or spherical in shape as shown In other embodiments, the bubbles 210 may be formed with a volumetric shape that is generally conical, cylindrical, tubular having a flattened or hemispherical end, ovoidal, pyramidal, cuboidal, or geodesic shape. The bubbles 210 that are generally hemispherical or spherical in shape may have a diameter between about 0.5 mm and 10 mm. The bubbles 210 also may have a pitch, i.e., the center to center distance between each of the bubbles 210, between about 1.5 mm and 15 mm. Because the sealed region 509 defines the base of the bubbles 210 including the diameter of a circular base and the pitch of adjacent bubbles 210, the surface area of the bubble manifold 202 covered by the bubbles 210 may also be determined as a percentage, i.e., the cell coverage percentage. In one example embodiment where the diameter of the bubbles 210 is about 1.0 mm and the pitch is about 2.0 mm, the bubble coverage is about 22% of the surface area of the bubble manifold 202. In another example embodiment where the diameter of the bubbles 210 is about 2.0 mm and the pitch is about 5.0 mm, the bubble coverage percentage is about 14% of the surface area of the bubble manifold 202. In yet another example embodiment where the diameter of the bubbles 210 is about 1.0 mm and the pitch is about 1.5 mm, the bubble coverage percentage is about 30% of the surface area of the bubble manifold 202. In still another example embodiment where the diameter of the bubbles 210 is about 1.5 mm, the pitch is about 2.0 mm, and the bubbles 210 are more tightly arranged such that there are about 28.5 bubbles in a 10 mm² section of the bubble manifold 202, the bubble coverage percentage is about 51% of the surface area of the bubble manifold 202. Depending on the diameter, pitch, and arrangement of the bubbles 210, the bubble coverage percentage may range between about 10% and about 55% of the surface area of the bubble manifold 202. Bubbles 210 having other base shapes or volumetric shapes also may have a bubble coverage percentage in generally the same range. In some embodiments, the bubbles 210 may be arranged in a staggered pattern (e.g. in offset rows), for example so as not to be aligned in a grid-like pattern.

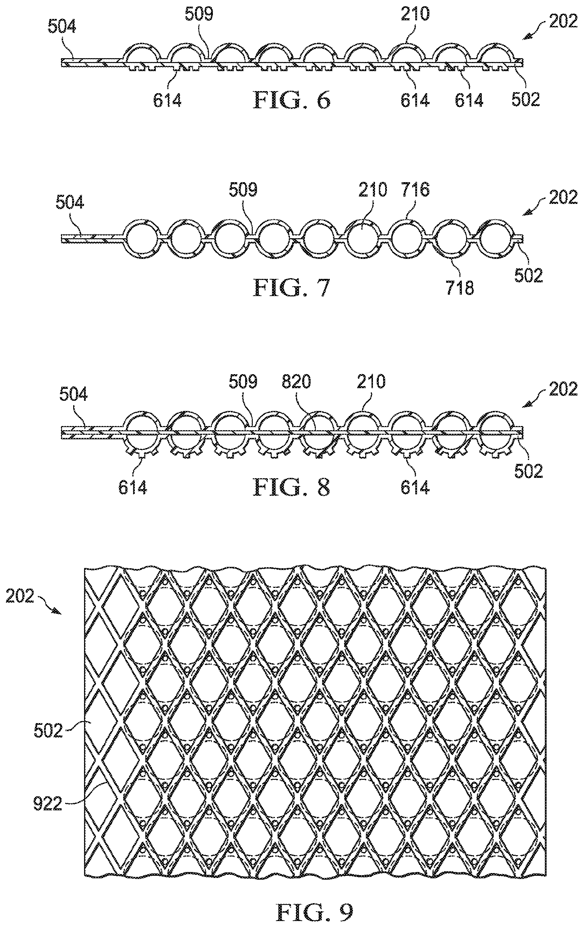

Some embodiments of the bubbles 210 may have three-dimensional shapes, including hemispherical shapes, spherical shapes, conical shapes, cylindrical shapes, pyramidal shapes, cuboidal shapes, ovoidal shapes, or tubular shapes formed with a flattened or hemispherical end. These shapes may be formed in one or both of the first layer 502 and the second layer 504, such as the single hemispherical shape shown in Figure 6 (bubbles 210) and the two hemispherical shapes that are aligned with one another to form a spherical shape as shown in Figure 7 (bubbles 210). The bubbles 210 may have a height between about 0.25 mm and about 5 mm, e.g., about half the diameter of bubbles 210 having a hemispherical shape as described in the examples above. In some embodiments, the bubbles 210 may measure about 10 mm in diameter and about 3 mm in height. In other example embodiments, the bubbles 210 may have a generally tubular shape formed with generally parallel walls extending from the sealed region 509 to a hemispherical end. In yet other example embodiments, bubbles 210 having a tubular shape may have a diameter of about 1.5 mm and an average height in a range between about 2.0 mm and 4.0 mm.

Still referring primarily to Figure 5, the first layer 502 and the second layer 504 may each have a thickness of about 5 µm to 500 µm, and the sealed region 509 may be between about 10 µm and 1000 µm in thickness. The walls of the bubbles 210, after being formed by coupling the first layer 502 and the second layer 504 together, may have a thickness relative to the thickness of the first layer 502 and the second layer 504 defined by a draw ratio, which is the ratio of the average height of the bubbles 210 to the average thickness of the first layer 502 and the second layer 504. In one example embodiment where the bubbles 210 have a generally tubular shape, the first layer 502 and the second layer 504 may have an average thickness of 250 µm and the bubbles 210 may have an average height in a range between about 2.0 mm and 4.0 mm with a diameter of about 1.5 mm. Consequently, the bubbles 210 have a draw ratio ranging from about 8:1 to about 16:1 for heights of 2.0 mm and 4.0 mm, respectively. In another example embodiment, the first layer 502 and the second layer 504 may have an average thickness of 100 µm and the bubbles 210 may have an average height in a range between about 2.0 mm and 4.0 mm with a diameter of about 1.5 mm. Consequently, the bubbles 210 have a draw ratio ranging from about 20:1 to about 40:1 for heights of 2.0 mm and 4.0 mm, respectively. In yet other example embodiments, it is desirable that the draw ratio be greater than about 16:1 where the thickness of the first layer 502 and the second layer 504 is less than about 250 µm. The first layer 502 and the second layer 504 may each have the same thickness or different thicknesses and flexibilities, but are substantially non-stretchable as desired above so that the bubbles 210 maintain a generally constant volume without bursting after negative pressure or instillation fluid is applied to the bubble manifold 202. Consequently, even when a load is applied to the bubble manifold 202 which squeezes bubbles 210 into a different shape, the bubbles 210 are sufficiently flexible to recover their original shape after being squeezed without bursting.

Figure 6 is a section view of an example embodiment of the bubble manifold 202 of Figure 5, illustrating additional details that may be associated with some embodiments. For example, the bubble manifold 202 of Figure 6 may be configured so that closed cells extend from only one side of the sealed region 509 of the bubble manifold 202, such as bubbles 210 formed of closed cells having a hemispherical shape. More specifically, the bubble manifold 202 may comprise two sheets of polymeric film, such a first layer 502 and a second layer 504, having inner surfaces coupled to each other in a pattern defining a plurality of bubbles 210. The first layer 502 and the second layer 504 may be sealed to each other in a sealed region 509 that defines the bubbles 210 that are generally hemispherical in shape. The bubbles 210 may be formed on only one side of the sealed region 509 by using sheets of polymeric film having a different thickness or flexibility. For example, the bubbles 210 may be formed in the second layer 504 by applying a vacuum to the second layer 504 where the first layer 502 is sufficiently thicker than the second layer 504 to withstand the vacuum being applied and retain a generally planar shape. The bubbles 210 having other shapes may be formed to extend from only one side of the sealed region 509 and may be formed by using a variety of different methods. For example, the shape of the bubbles 210 may be formed separately in the second layer 504, which can be subsequently coupled to the first layer 502 to complete the encapsulation of the bubbles 210. The first layer 502 may have the same thickness as the second layer 504 so that the sealed region 509 remains thin and flexible.

Still referring primarily to Figure 6, in some embodiments, the bubble manifold 202 may further include textured surface features on one or more surfaces of either or both of the first layer 502 and the second layer 504. The textured surface features may be included on a surface of either or both of the first layer 502 and the second layer 504 that may be placed facing the tissue site. The textured surface features may be protrusions or indentations for enhancing fluid flow through the bubble manifold 202 and to increase micro-strains against the tissue site for enhancing granulation. More specifically, the textured surface features may include a pattern of individual nodes or projections embossed on the outer surface of the first layer 502 and/or second layer 504, a grid embossed on the outer surface of the first layer 502 and/or second layer 504, a pattern or grid of grooves formed into the outer surface of the first layer 502 and/or second layer 504, or any combination of the foregoing. For example, as shown in Figure 6, the bubble manifold 202 may include textured surface features in the form of nodes 614, which may be embossed on the outer surface of the first layer 502 so that the nodes 614 contact the tissue site when the bubble manifold 202 is positioned at the tissue site.

The nodes 614 may be projections that are flexible or rigid. In some embodiments, the projections may be formed from a substantially gas-impermeable material such as silicone. In other embodiments, the projections may be formed from a semi-gas-permeable material. The projections may be formed as an integral part of the first layer 502 and the second layer 504, and they may also be formed from the same material as the first layer 502 and the second layer 504. In some embodiments, the projections may be solid, while in other embodiments, the projections may be hollow to increase flexibility. The projections may form a plurality of channels and/or voids as described below to distribute negative pressure and allow for fluid flow among the projections. The projections may be dimensioned to provide local load points at a tissue site sufficient to create micro-strains at the tissue site for stimulating granulation formation when negative pressure is applied. The pattern and position of the projections may be uniform or non-uniform. The projections may have different shapes including, for example, the shape of a spike, cone, pyramid, dome, cylinder, or rectangle. The shapes of the projections may be uniform or non-uniform depending on the tissue site. The shapes of the projections may occupy a volume defined by a cube volume where the side of the cube would range from approximately 0.2 mm to approximately 1.5 mm. In one embodiment, the spike shape may have a base width or diameter of about 0.2 mm and a vertical height of between about 0.4 mm and 0.8 mm. In another embodiment, the cone shape may have a base diameter of about 0.4 mm and a vertical height of between 0.4 mm and 1.2 mm. In yet another embodiment, the dome shape may have a spherical cap or parabolic shape with a base diameter ranging from about 0.4 mm to 1 mm.

Figure 7 is a section view of another example of the bubble manifold 202 of Figure 5, illustrating additional details that may be associated with some embodiments. For example, the bubble manifold 202 of Figure 7 may include bubbles 210 comprising portions of closed cells that are formed in both of the two sheets or layers of the bubble manifold 202, so that the portions of bubbles 210 extend from both sides of the sealed region of the bubble manifold 202. More specifically, the bubble manifold 202 may comprise two sheets of polymeric film, first layer 502 and second layer 504, having inner surfaces coupled to each other in a pattern defining a plurality of bubbles 210. For example, the portions of bubbles 210 formed in each of the first layer 502 and the second layer 504 may each be hemispherical in shape, such as hemispherical cell 716 and hemispherical cell 718. The hemispherical cell 716 and hemispherical cell 718 may then be aligned to form a single bubble 210 in the form of a closed cell having a generally spherical shape In other words, each of the single bubbles 210 comprises two hemispherical cells, hemispherical cell 716 and hemispherical cell 718, formed in the second layer 504 and the first layer 502, respectively. The first layer 502 and the second layer 504 may be sealed to each other in a sealed region 509 that defines the bubbles 210 that are generally spherical in shape. In other example embodiments, the closed cells in each sheet may not be aligned with each other, but rather are overlapped or aligned with the sealed portion of the opposite sheet. The bubbles 210 may be formed on both sides of the sealed region 509 by using sheets of polymeric film having a different thickness or flexibility. For example, the shape of the bubbles 210 may be asymmetric when the first layer 502 and the second layer 504 have different thicknesses or flexibilities from each other. However, when the first layer 502 and the second layer 504 have substantially identical thickness or flexibility, the shape of the bubbles 210 may be substantially spherical, as shown in Figure 7.

Figure 8 is a section view of another example of the bubble manifold 202 of Figure 5, illustrating additional details that may be associated with some embodiments. For example, the bubble manifold 202 of Figure 8 may include a third sheet forming a multi-sheet configuration where the third sheet is disposed between a first sheet and a second sheet to form closed cells that may be generally spherical in shape formed by two hemispherical sections separated by portions of the third sheet of material. For example, the bubble manifold 202 of Figure 8 may include a first layer 502 and a second layer 504 of polymeric film having inner surfaces coupled or bonded to a third layer 820 to form sealed region 509 defining a plurality of bubbles 210 in the form of closed cells. The bubbles 210 are generally spherical in shape and formed by two hemispherical sections that are separated by portions of the third layer 820. The first layer 502 and the second layer 504 may be coupled or bonded to the third layer 820 using a variety of different methods including, for example, melting (e.g., RF, ultrasonic, and heat), adhesives using both hot melt and solvents, and pressing techniques. The third layer 820 may be formed from a polymeric film, and may also be perforated to permit airflow between the two hemispherical sections of the bubbles 210. In some embodiments, the third layer 820 may comprise the same material as the first layer 502 and/or the second layer 504. When the third layer 820 is formed from a polymeric material, the third layer 820 may also be textured to provide wicking capability. The third layer 820 may also be formed from a polyester material to provide wicking within the bubbles 210 and may further include fibers flocked into the polyester material to provide additional wicking capability. The third layer 820 may also include an antimicrobial layer or antimicrobials coated on the third layer 820.

In some embodiments, the bubble manifold 202 of Figure 8 may also include textured surface features on one or more surfaces of either or both of the first layer 502 and the second layer 504. The textured surface features may be protrusions or indentations, as discussed with respect to the textured surface features of Figure 6. For example, the bubble manifold 202 may include projections or nodes 614 embossed on the outer surface of the first layer 502 and, more specifically, on the surface of the bubbles 210 so that the nodes 614 contact the tissue site if the bubble manifold 202 is positioned at the tissue site.

Figure 9 is a schematic view of a portion of another example of the bubble manifold 202 of Figure 2, illustrating additional details that may be associated with some embodiments For example, a number of different textures or shapes may be formed on the outside surface of a first layer 502 that may be flat and may be for facing a tissue site when in use. In one exemplary embodiment, a grid 922 may be embossed or extruded in a woven pattern on the outer surface of the first layer 502. The pattern of the grid 922 may have a variety of shapes, like the diamond-shaped pattern shown. It should be understood that many types of protrusions or grids may be formed on a surface of the first layer 502 or a first sheet of other disclosed embodiments of the bubble manifold 202 for enhancing fluid flow through or along the bubble manifold 202 and/or enhance granulation of a tissue site. Moreover, it should be understood that any of such protrusions or grids may be formed by embossing, welding, or any other similar type of coupling mechanism.

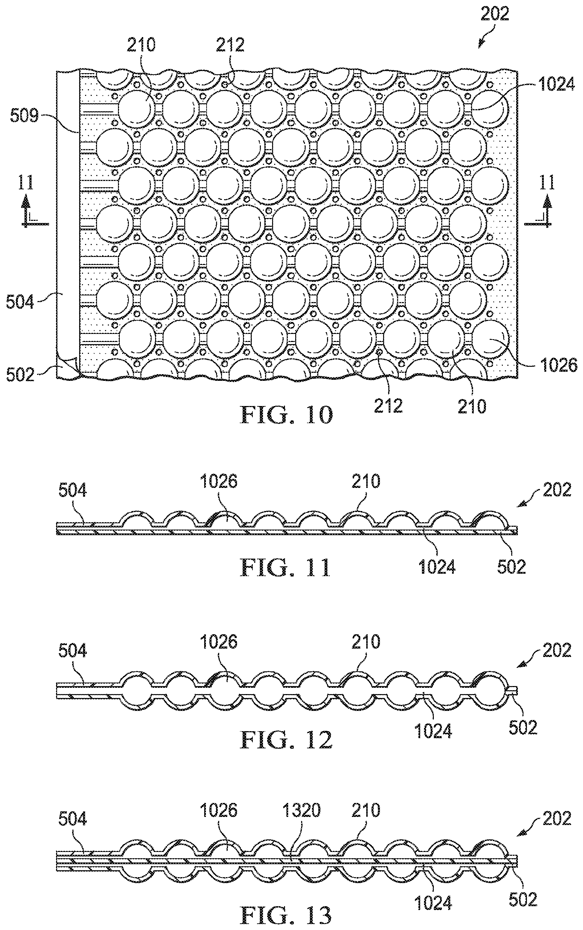

Figure 10 is a schematic view of a portion of another example of the bubble manifold 202 of Figure 2, illustrating additional details that may be associated with some embodiments. The bubble manifold 202 may be similar to the embodiments of the bubble manifold 202 previously discussed but may further include chambers formed by interconnected closed cells to better distribute the apposition force applied to the bubble manifold 202 as a result of the application of negative pressure since the volume of the chambers is greater than the volume of the individual closed cells. In one embodiment, as shown in Figure 10, the bubble manifold 202 includes a first layer 502 and a second layer 504, each of which may be a polymeric film having inner surfaces coupled to each other in a pattern forming a sealed region 509 defining a plurality of bubbles 210 comprising closed cells. The sealed region 509 may also be perforated to provide pathways for fluid to flow through the bubble manifold 202. In one exemplary embodiment, the sealed region 509 may comprise a plurality of apertures 212 that are formed between the bubbles 210 in the sealed region 509 that extend through both of the first layer 502 and the second layer 504 to permit fluid flow through the bubble manifold 202. The bubble manifold 202 may also comprise a plurality of passageways 1024 fluidly coupling at least two of the bubbles 210 to form a closed chamber. In one exemplary embodiment, a closed chamber 1026 is formed by all of the bubbles 210 in a row fluidly coupled by the passageways 1024 as shown in Figure 10. Closed chambers 1026 may be formed in each of the other six rows as also shown in Figure 10. The formation of closed chambers with closed cells in any pattern may distribute apposition forces applied to the bubble manifold 202 more equally across the bubble manifold 202.

Figure 11 is a section view of an example embodiment of the bubble manifold 202 of Figure 10, illustrating additional details that may be associated with some embodiments. For example, the bubble manifold 202 of Figure 11 may include two sheets of polymeric film, first layer 502 and second layer 504, having inner surfaces coupled to each other in a pattern defining a plurality of bubbles 210 comprising closed cells. The first layer 502 and the second layer 504 may be sealed to each other in a sealed region that defines the bubbles 210 that are generally hemispherical in shape. The bubble manifold 202 also may comprise a plurality of passageways 1024 interconnecting the bubbles 210 to form a closed chamber 1026. The closed chamber 1026 may be formed in only one of the first layer 502 and the second layer 504 so that they extend from only one side of the sealed region, as shown in Figure 11.

Figure 12 is a section view of another example of the bubble manifold 202 of Figure 10, illustrating additional details that may be associated with some embodiments. For example, the bubble manifold 202 of Figure 12 may include two sheets of polymeric film, the first layer 502 and the second layer 504, having inner surfaces coupled to each other in a pattern defining a plurality of bubbles 210 comprising closed cells. The first layer 502 and the second layer 504 may be sealed to each other in a sealed region that defines the bubbles 210 that are generally spherical in shape. The bubble manifold 202 also may comprise a plurality of passageways 1024 interconnecting the bubbles 210 to form a closed chamber 1026. The closed chamber 1026 is formed in both of the first layer 502 and the second layer 504 so that they extend from both sides of the sealed region that provides more flexibility and cushioning than the closed chamber 1026 of Figure 11 extending from only one side of the sealed region.

Figure 13 is a section view of another example of the bubble manifold 202 of Figure 10, illustrating additional details that may be associated with some embodiments. For example, the bubble manifold 202 of Figure 13 may include two sheets of polymeric film, first layer 502 and second layer 504, having inner surfaces coupled or bonded to a third layer 1320 to form a sealed region defining a plurality of bubbles 210 comprising closed cells. The bubbles 210 are generally spherical in shape and formed by two hemispherical sections that are separated by portions of the third layer 1320. The first layer 502 and the second layer 504 may be coupled or bonded to the third layer 1320 using a variety of different methods including, for example, melting (e.g., RF, ultrasonic, and heat), adhesives using both hot melt and solvents, and pressing techniques. The third layer 1320 may be formed from a polymeric film and may also be perforated to permit airflow between the two hemispherical sections of the bubbles 210. When the third layer 1320 is formed from a polymeric material, the third layer 1320 may also be textured to provide wicking capability. The third layer 1320 may also be formed from a polyester material to provide wicking within the bubbles 210, and may further include fibers flocked into the polyester material to provide additional wicking capability. The third layer 1320 also may include an antimicrobial layer or antimicrobials coated on the third layer 1320. The third layer 1320 of Figure 13 may also provide a plurality of passageways 1024 interconnecting the bubbles 210 to form a closed chamber 1026. The closed chamber 1026 of Figure 13 is formed in both of the first layer 502 and the second layer 504 so that they extend from both sides of the sealed region.

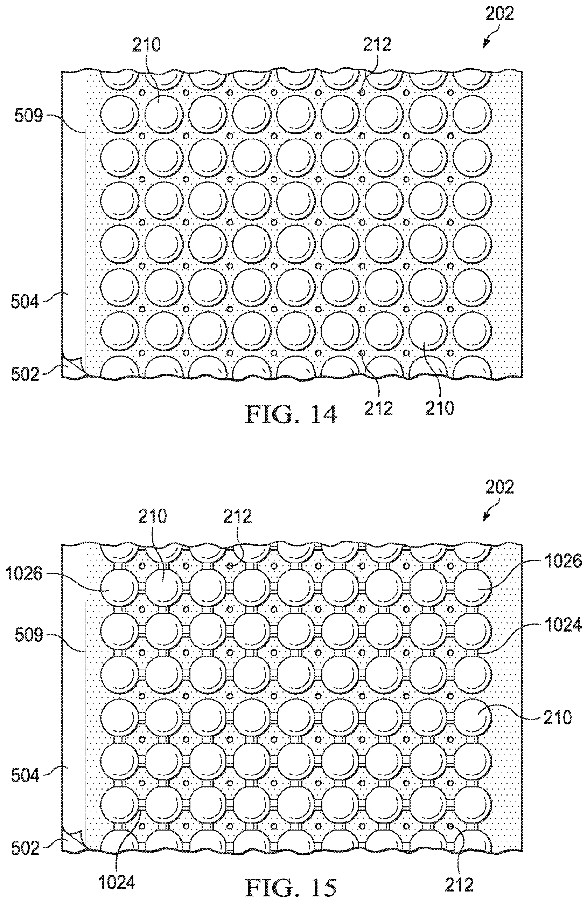

Figure 14 is a schematic view of a portion of another example of the bubble manifold 202 of Figure 2, illustrating additional details that may be associated with some embodiments. The bubble manifold 202 may be similar to the embodiments of the bubble manifold 202 previously discussed but may include different arrangements of bubbles 210 comprising closed cells, which may be suitable for particular forms of therapy being utilized. Previously discussed with respect to Figure 5 were bubbles 210 comprising closed cells which are in a staggered arrangement so that the individual cells may be more closely nested together between the alternating rows to form a nested pattern of cells formed on the same plane as defined by the sealed region 509. Figure 14 depicts a bubble manifold 202 that may include two sheets of polymeric film, first layer 502 and second layer 504, having inner surfaces sealed to each other in a pattern defining a plurality of bubbles 210 comprising closed cells in close proximity to one another. However, the rows and columns of bubbles 210 of Figure 14 are not staggered, but rather arranged in an aligned pattern. Depending on the diameter and pitch of the bubbles 210, the cell coverage percentage may range between about 10% and about 55% of the surface area of the bubble manifold 202. The first layer 502 and second layer 504 may be sealed to each other in a sealed region 509 that defines the bubbles 210. In this embodiment, the rows and columns of the bubbles 210 are arranged in line to form an aligned pattern. The bubble manifold 202 may also include a sealed region 509 that may be perforated as described above. In some embodiments, the sealed region 509 may include a plurality of apertures 212 between the bubbles 210 and extending through both the first layer 502 and the second layer 504 to permit fluid to flow through the bubble manifold 202. The apertures 212 may have similar dimensions as apertures 212 of Figure 5. The pattern of bubbles 210 may have a variety of arrangements.

Figure 15 is a schematic view of a portion of another example of the bubble manifold 202 of Figure 2, illustrating additional details that may be associated with some embodiments. The bubble manifold 202 may be similar to the bubble manifold 202 of Figure 14 and may include two sheets of polymeric film, first layer 502 and second layer 504, having inner surfaces sealed to each other in a pattern defining a plurality of bubbles 210 comprising closed cells in close proximity to one another. The first layer 502 and the second layer 504 may be sealed to each other in a sealed region 509 that defines the bubbles 210. The sealed region 509 may also be perforated to provide pathways for fluid to flow through the bubble manifold 202. In one exemplary embodiment, the sealed region 509 may comprise a plurality of apertures 212 that are formed between the bubbles 210 in the sealed region 509 that extend through both of the first layer 502 and the second layer 504 to permit fluid flow through the bubble manifold 202. The bubble manifold 202 may also comprise a plurality of passageways 1024 interconnecting the bubbles 210 to form a closed chamber 1026. In one exemplary embodiment, a closed chamber 1026 is formed by all of the bubbles 210 in a row fluidly coupled by the passageways 1024 as shown in Figure 15. Closed chambers 1026 may be formed in each of the other six rows, or formed to include multiple rows, as also shown in Figure 15. The formation of closed chambers 1026 with bubbles 210 in any pattern may distribute apposition forces applied to the bubble manifold 202 more equally across the bubble manifold 202 as opposed to a layer having only closed cells.

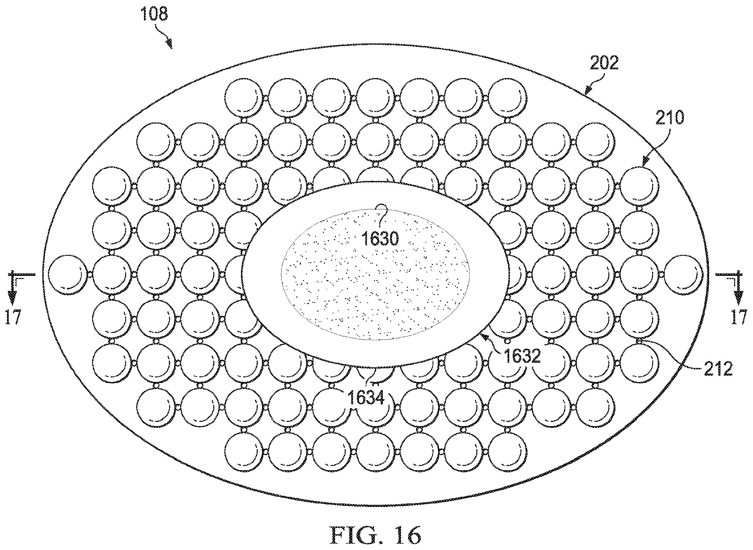

Figure 16 is a schematic top-view of another example of a tissue interface 108, illustrating additional details that may be associated with some embodiments. For example, the tissue interface 108 of Figure 16 may comprise a manifold and may be adapted to provide negative pressure from the negative-pressure source 104 of the therapy system 100 to a tissue site, and to transport fluid extracted from the tissue site. The tissue interface 108 may comprise a bubble manifold 202 and a foam manifold 1630. The bubble manifold 202 may comprise a layer of liquid-impermeable material having a plurality of bubbles 210, which may be in the form of open-celled blisters, spacers, protrusions, or closed cells. The bubble manifold 202 of the tissue interface 108 of Figure 16 may additionally comprise apertures 212 positioned between the bubbles 210 to allow fluid transfer through the bubble manifold 202 and tissue interface 108.

The tissue interface 108 of Figure 16 may also comprise foam manifold 1630, which may include a portion of manifolding or filler material positioned in a central portion of the tissue interface 108. For example, the tissue interface 108 may include the foam manifold 1630, which may be positioned adjacent a central portion of a surface of the bubble manifold 202 of the tissue interface 108. As shown in Figure 16, the foam manifold 1630 may have a circular or disc shape; however, other embodiments of the tissue interface 108 may also include a foam manifold 1630 of a variety of shapes, such as square or box shapes, triangular or pyramidal shapes, or any other suitable shape or configuration. In some alternative embodiments, the foam manifold 1630 may be positioned against a center region of the bubble manifold 202 that includes at least one film of liquid-impermeable material, but does not include bubbles.

The foam manifold 1630 may include one or more types of manifolding materials. For example, the foam manifold 1630 may be a porous material having interconnected fluid pathways. Examples of suitable porous material that can be adapted to form interconnected fluid pathways (e.g., channels) may include cellular foam, including open-cell foam such as reticulated foam, as well as other porous material such as gauze or felted mat that generally include pores. Liquids, gels, and other foams may also include or be cured to include apertures and fluid pathways. In some examples, the foam manifold 1630 may be reticulated polyurethane foam such as found in GRANUFOAM^{™} Dressing or V.A.C. VERAFLO^{™} Dressing, both available from Kinetic Concepts, Inc. of San Antonio, Texas.

In some embodiments, the tissue interface 108 of Figure 16 may further include a film material, such as film 1632, for positioning over the foam manifold 1630. The film 1632 may comprise any type of film material, such as any of the suitable non-porous or liquid-impermeable polymeric or drape materials. The film 1632 may further include a plurality of fenestrations, which may be suited for allowing fluid communication between a space external to the tissue interface 108 and the foam manifold 1630. In some embodiments, the film 1632 may be sealed around the foam manifold 1630 via a weld 1634 between the film 1632 and a surface of the bubble manifold 202 surrounding a perimeter or circumference of the foam manifold 1630.

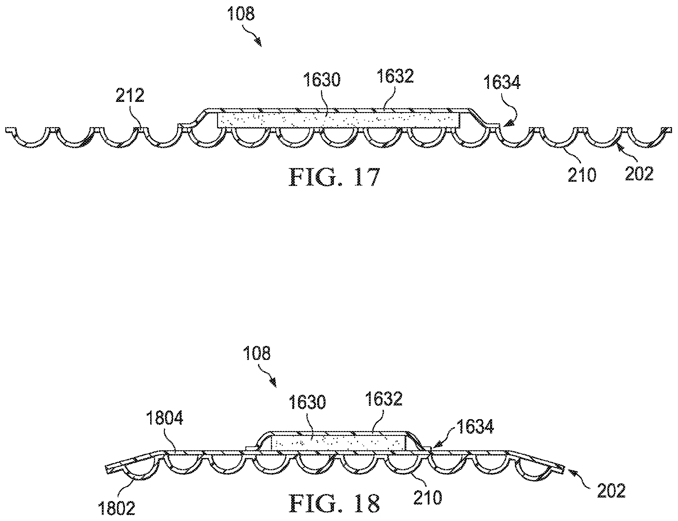

Figure 17 is a section view of an example embodiment of the tissue interface 108 of Figure 16, illustrating additional details that may be associated with some embodiments. For example, the tissue interface 108 may include bubble manifold 202, which may be formed of a single sheet or film of liquid-impermeable material comprising bubbles 210 and apertures 212 formed thereon. In some embodiments, the bubbles 210 may be in the form of blisters according to the principles previously discussed. As shown in Figure 17, the apertures 212 may be formed in the portions of the bubble manifold 202 that are between the bubbles 210 and may extend through the bubble manifold 202 to permit fluids to flow through the tissue interface 108. The number of apertures 212 may vary depending on the type of negative pressure and instillation therapy to be provided by the therapy system 100. The foam manifold 1630 may be placed on a surface of the bubble manifold 202, with the film 1632 applied over and around the foam manifold 1630. The film 1632 may be welded to a surface of the bubble manifold 202 via weld 1634. As shown in Figure 17, the bubble manifold 202 may be positioned so that the bubbles 210, which may be in the form of blisters, protrude, extend, or face downward so as configured to face a tissue site. However, in alternative embodiments, the bubble manifold 202 may be reversed, so that the bubbles 210 extend or face upwards towards the foam manifold 1630.

Figure 18 shows a section view of another example embodiment of the tissue interface 108 of Figure 16, illustrating some additional details. For example, the tissue interface 108 of Figure 18 may comprise a bubble manifold 202, which may be formed from a plurality of liquid-impermeable layers. The tissue interface 108 of Figure 18 may also comprise foam manifold 1630. In some embodiments, the bubble manifold 202 of the tissue interface 108 of Figure 18 may include a first layer of liquid-impermeable film, first layer 1802, and a second layer of liquid-impermeable film, second layer 1804. First layer 1802 and second layer 1804 may be sealingly coupled to one another in any suitable manner. As previously discussed with respect to other embodiments, the first layer 1802 and the second layer 1804 may comprise a non-adherent material, such as a breathable polyurethane film material.

The bubble manifold 202 of the tissue interface 108 of Figure 18 may include a plurality of bubbles 210, which may be in the form of closed cells. For example, the bubbles 210 may comprise closed cells formed between the first layer 1802 and the second layer 1804 and may be filled with fluid such as air. Similar to embodiments previously discussed, each of the first layer 1802 and the second layer 1804 may have inner surfaces coupled to each other to form a sealed region defining a plurality of closed cells forming the bubbles 210. In the example embodiment of the tissue interface 108 of Figure 18, the bubbles 210 may be formed between a majority or substantially all of the surface areas of the inner surfaces of the first layer 1802 and the second layer 1804. The bubble manifold 202 may additionally comprise apertures through the first layer 1802 and the second layer 1804, and between the bubbles 210, to allow fluid transfer through the bubble manifold 202 of the tissue interface 108.

As shown in Figure 18, the tissue interface 108 may be configured so that the bubbles 210 comprising closed cells extend from only one side of the bubble manifold 202, which in this instance may be configured to face a tissue site. The bubbles 210 of Figure 18 are shown with the first layer 1802 of the bubble manifold 202 forming hemispherical-shaped closed cells and the second layer 1804 of the bubble manifold 202 retaining a generally planar shape.

The tissue interface 108 of Figure 18 may also include a portion of manifolding or filler material positioned in a central portion of the tissue interface 108. For example, the tissue interface 108 may include foam manifold 1630, which may be positioned adjacent a central portion of an external surface of the second layer 1804 of the bubble manifold 202. In some alternative embodiments, the foam manifold 1630 may be positioned in a center region of the tissue interface 108 that includes portions of the first layer 1802 and second layer 1804 of the bubble manifold 202, but does not include bubbles. In such embodiments, a center portion of the first layer 1802 and second layer 1804 of the bubble manifold 202 corresponding to the foam manifold 1630 may have inner surfaces sealed to each other, without any bubbles between the first layer 1802 and second layer 1804.

In some embodiments, the tissue interface 108 of Figure 18 may further include a film material, such as film 1632, for positioning over the foam manifold 1630. For example, the foam manifold 1630 may be positioned between an external surface of the second layer 1804 of the bubble manifold 202 and the film 1632. The film 1632 may further include a plurality of fenestrations. The film 1632 may be sealed around the foam manifold 1630 via a weld 1634 between the film 1632 and an external surface of the second layer 1804 of the bubble manifold 202 surrounding a perimeter or circumference of the foam manifold 1630.

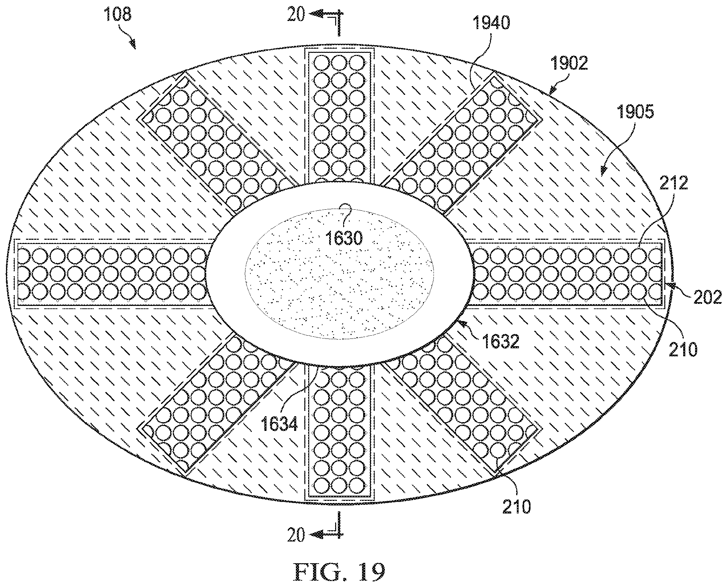

Figure 19 is a schematic top-view of another example embodiment of a tissue interface 108, illustrating additional details that may be associated with some embodiments. The tissue interface 108 of Figure 19 may comprise a manifold and may be adapted to provide negative pressure from the negative-pressure source 104 of the therapy system 100 to a tissue site, and to transport fluid extracted from the tissue site. The tissue interface 108 may comprise a fenestrated manifold 1902, a bubble manifold 202, and a foam manifold 1630. The fenestrated manifold 1902 may comprise a liquid-impermeable layer having fenestrations 1905.

The bubble manifold 202 may include a central portion and one or more fluid channels 1940, which may extend radially away from the central portion and may be configured to provide enhanced manifolding to the tissue interface 108. For example, the fluid channels 1940 of Figure 19 may enhance the distribution of negative pressure from the negative-pressure source 104 of the therapy system 100 as well as enhance fluid removal capability of the tissue interface 108 when applied to a tissue site. Each of the fluid channels 1940 may comprise a plurality of bubbles 210, which may be in the form of open-celled blisters, spacers, protrusions, or closed cells. The width of each of the fluid channels 1940 may vary; however, in some instances, the fluid channels 1940 may be between about 2 cm and 15 cm in width. In some embodiments of the tissue interface 108 where the fluid channels 1940 include bubbles 210 comprising open-celled blisters, the bubble manifold 202 may be in the form of a single layer of liquid-impermeable material. In some embodiments, the bubble manifold 202 may be configured so that the bubbles 210 protrude or extend towards the fenestrated manifold 1902, while in other embodiments, the bubbles 210 may protrude or extend upwards or away from the fenestrated manifold 1902. The fluid channels 1940 may additionally comprise apertures 212 positioned between the bubbles 210 to allow fluid transfer through the bubble manifold 202 of the tissue interface 108.

The tissue interface 108 of Figure 19 may also comprise foam manifold 1630, which may include a portion of manifolding or filler material positioned in a central portion of the tissue interface 108. For example, the tissue interface 108 may include a foam manifold 1630, which may be positioned adjacent a central portion of a surface of the bubble manifold 202 of the tissue interface 108. As shown in Figure 19, the foam manifold 1630 may assume a circular or disc shape, as well as other possible shapes. Depending on the particular embodiment, the foam manifold 1630 may be positioned against a center region of the bubble manifold 202 that includes at least one film of liquid-impermeable material, which may or may not include bubbles in the center region. Similar to the previous embodiments discussed, the foam manifold 1630 may include one or more types of manifolding materials, such as an open-cell foam. The tissue interface 108 may further include a film 1632 positioned over the foam manifold 1630, and the foam manifold 1630 may be positioned between a surface of the bubble manifold 202 and the film 1632. The film 1632 may comprise any type of film material such as any suitable liquid-impermeable polymeric or drape materials, and may further include fenestrations. The film 1632 may be sealed around the foam manifold 1630 via a weld 1634 between the film 1632 and a surface of the bubble manifold 202 and/or fenestrated manifold 1902 surrounding a perimeter or circumference of the foam manifold 1630.

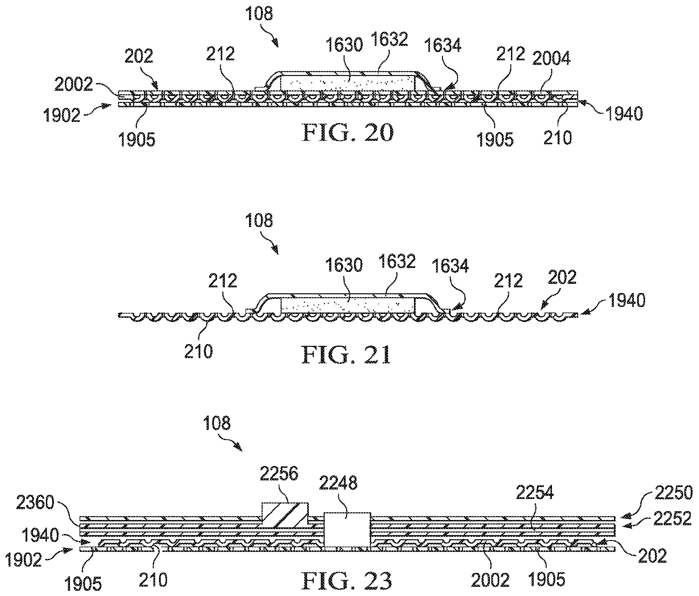

Figure 20 is a section view of an example embodiment of the tissue interface 108 of Figure 19, illustrating additional details that may be associated with some embodiments. In some embodiments, the tissue interface 108 may include a fenestrated manifold 1902, a bubble manifold 202, and a foam manifold 1630. The fenestrated manifold 1902 may comprise a liquid-impermeable layer having fenestrations 1905. The bubble manifold 202 of Figure 20 may be formed from a plurality of liquid-impermeable layers. For example, the bubble manifold 202 may include a first layer of liquid-impermeable film, such as first layer 2002, and a second layer of liquid-impermeable film, such as second layer 2004 First layer 2002 and second layer 2004 may be sealingly coupled to one another in any suitable manner. As previously discussed with respect to other embodiments, the first layer 2002 and the second layer 2004 may comprise a non-adherent material, such as a breathable polyurethane film material. Each of the first layer 2002 and the second layer 2004 may include apertures, such as apertures 212, for allowing fluid transfer through the first layer 2002 and the second layer 2004 of the bubble manifold 202. The bubble manifold 202 of Figure 20 may include one or more fluid channels 1940, which may be formed from portions of the first layer 2002 and the second layer 2004 that have been welded together to form the fluid channels 1940.

In some embodiments, the fluid channels 1940 may include a plurality of bubbles 210 comprising closed cells. For example, bubbles 210 comprising closed cells may extend along the length of each of the fluid channels 1940. The bubbles 210 may assist with communicating negative pressure to a tissue site and drawing fluids through the fluid channels 1940. For example, the bubbles 210 comprising closed cells may help maintain open fluid pathways through the fluid channels 1940 for receiving negative pressure from the negative-pressure source 104 and to distribute negative pressure through the multiple apertures 212 or perforations along the fluid channels 1940 and across a tissue site, which may have the effect of collecting fluids from across the tissue site and drawing the fluids toward the negative-pressure source 104.

The bubbles 210 comprising closed cells of the fluid channels 1940 may be formed by or between the first layer 2002 and the second layer 2004, and the closed cells may be filled with fluid such as air. Within the fluid channels 1940, each of the first layer 2002 and the second layer 2004 may comprise or consist essentially of a polymer film having inner surfaces coupled to each other to form one or more sealed regions. The bubbles 210 may be formed between the surface areas of the inner surfaces of the first layer 2002 and the second layer 2004 in the portions of the sealed regions defining the fluid channels 1940, and in this example embodiment, the bubbles 210 may be extending or protruding downwards as portions of the first layer 2002. The bubble manifold 202 may additionally include apertures 212 along the fluid channels 1940 through the first layer 2002 and the second layer 2004, and between the bubbles 210, to allow fluid transfer through the portions of the first layer 2002 and second layer 2004 forming the fluid channels 1940 of the bubble manifold 202. The fluid channels 1940 may provide enhanced manifolding or fluid transport capability along the surface of the first layer 2002 due to the presence of the bubbles 210 and associated spaces between the bubbles 210.

Figure 21 shows a section view of another example embodiment of the tissue interface 108 of Figure 19, illustrating additional details that may be associated with some embodiments. As shown in Figure 21, in some alternative embodiments of the tissue interface 108 of Figure 19, the fenestrated manifold 1902 may be omitted. In such embodiments, the bubble manifold 202 may instead include a layer of liquid-impermeable material defining the size and shape of the tissue interface 108. The single layer of liquid-impermeable material of the bubble manifold 202 may include regions comprising the fluid channels 1940 as well as regions of the liquid-impermeable layer between the fluid channels 1940, which may have fenestrations. In such embodiments, the bubbles 210 may be formed in the regions of the bubble manifold 202 comprising the fluid channels 1940, and the bubbles 210 may face downward away from the foam manifold 1630 and towards a bottom surface of the tissue interface 108 so as to be placed in contact with a tissue site. The bubbles 210 may comprise open-celled blisters. The fluid channels 1940 of the embodiment of the tissue interface 108 of Figure 21 may provide substantially the same functionality as the fluid channels 1940 of Figure 20.

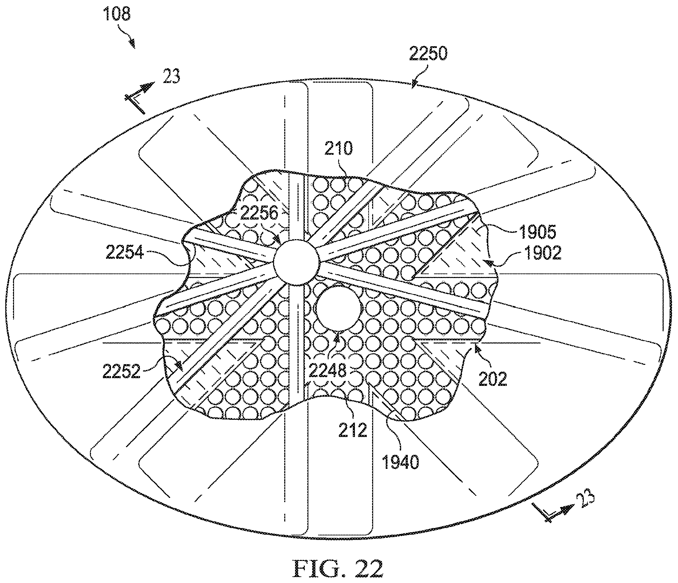

Figure 22 is a schematic cut-away view of another example embodiment of a tissue interface 108, illustrating additional details that may be associated with some embodiments. The tissue interface 108 of Figure 22 may comprise a manifold and may be adapted to facilitate both fluid removal from a tissue site as well as delivery of treatment fluids to the tissue site. For example, the tissue interface 108 of Figure 22 may be adapted to deliver negative pressure from the negative-pressure source 104 to a tissue site and to transport fluid extracted from the tissue site. The tissue interface 108 may also be adapted to deliver a fluid, such as a treatment fluid or medicament, from a fluid source, such as solution source 114 of therapy system 100, to the tissue site. The tissue interface 108 of Figure 22 may comprise a fenestrated manifold 1902 and a bubble manifold 202. The fenestrated manifold 1902 may comprise a liquid-impermeable layer having fenestrations 1905.

The bubble manifold 202 may include one or more fluid channels 1940, which may be configured to provide enhanced manifolding to the tissue interface 108. Each of the fluid channels 1940 may comprise a plurality of bubbles 210, which may be in the form of open-celled blisters, spacers, protrusions, or closed cells. In some embodiments of the tissue interface 108 where the fluid channels 1940 include bubbles 210 comprising open-celled blisters, the bubble manifold 202 may be in the form of a single layer of liquid-impermeable material. For example, the bubbles 210 along the fluid channels 1940 may be open-celled blisters that may be extending downwards towards the fenestrated manifold 1902. Alternatively, the bubbles 210 may extend upwards, or away from the fenestrated manifold 1902. The fluid channels 1940 may additionally comprise apertures 212 positioned between the bubbles 210 to allow fluid transfer through the bubble manifold 202 of the tissue interface 108. Additionally, the tissue interface 108 may include a fluid removal hub 2248, which may be in fluid communication with each of the fluid channels 1940 and may serve as a distribution mechanism for communicating negative pressure to each of the fluid channels 1940 from the negative-pressure source 104 of the therapy system 100. Fluids, such as wound exudates, from the tissue site may be removed along or through the fluid channels 1940 and the fluid removal hub 2248.

In some additional embodiments, the fluid channels 1940 of the tissue interface 108 may include a plurality of bubbles 210 comprising closed cells, which may be positioned along the length of each of the fluid channels 1940. In such embodiments, the bubble manifold 202 may include both a first liquid-impermeable layer and a second liquid-impermeable layer, and the bubbles 210 comprising closed cells may be formed by or between the first and second layers of the bubble manifold 202. The closed cells may be filled with fluid such as air. Apertures 212 may be included along the fluid channels 1940 through the first and second layers of the bubble manifold 202 and between the bubbles 210 for allowing fluid transfer through the portions of the first and second layers of the bubble manifold 202 forming the fluid channels 1940.

The tissue interface 108 of Figure 22 may also include a fluid distribution matrix 2252 for administering instillation fluid, such as from the solution source 114 of the therapy system 100, to a tissue site. The fluid distribution matrix 2252 may include a plurality of fluid delivery conduits 2254 and a fluid distribution hub 2256. The tissue interface 108 of Figure 22 may further include an instillation layer 2250, which may comprise at least one additional liquid-impermeable film, which may comprise fenestrations, that is positioned above an upper surface of the bubble manifold 202, and may be coextensive with the fenestrated manifold 1902. The bubble manifold 202 may be positioned between the instillation layer 2250 and the fenestrated manifold 1902.

In some embodiments, the fluid delivery conduits 2254 may include segments of tubing or other material for forming fluid conduits. The components of the fluid distribution matrix 2252 may be constructed of a variety of different materials. For example, some or all of the components of the fluid distribution matrix 2252 may be constructed of soft, medical-grade silicone or PVC tubing material. The plurality of fluid delivery conduits 2254 may vary in size, based on the particular size and application of the tissue interface 108, as well as the conditions of a tissue site to which the tissue interface 108 may be applied. For example, the fluid delivery conduits 2254 may include segments of tubing forming fluid conduits, and the tubing may have an inner diameter of between 0.5 mm and 5 mm. In some further embodiments, the fluid delivery conduits 2254 may be formed by welding or otherwise adhering portions of the instillation layer 2250 to a surface of the fenestrated manifold 1902 to form channels.

Figure 23 is a section view of an example embodiment of the tissue interface 108 of Figure 22, illustrating some additional details that may be associated with some embodiments. In the example embodiment of Figure 23, the tissue interface 108 may include a fenestrated manifold 1902 and a bubble manifold 202. The fenestrated manifold 1902 may comprise a liquid-impermeable layer having fenestrations 1905. The bubble manifold 202 may comprise a single layer of liquid-impermeable material and may include a plurality of bubbles 210 in the form of downward-facing open-celled blisters. In other embodiments, the bubble manifold 202 may include a first layer and a second layer that may be sealingly coupled to one another in any suitable manner to form bubbles comprising closed cells, and may include apertures. The bubble manifold 202 of Figure 23 may include one or more fluid channels 1940, which may be formed from the layer of liquid-impermeable material. In other embodiments, the fluid channels may be formed from a first layer and a second layer of liquid-impermeable material that have been welded together to form the fluid channels 1940. Additionally, the tissue interface 108 of Figure 23 may include a fluid distribution matrix 2252 and an instillation layer 2250.

In some embodiments, the fluid distribution matrix 2252 may be substantially encapsulated between the bubble manifold 202 and the instillation layer 2250. For example, the fluid distribution matrix 2252 may include fluid delivery conduits 2254, which may be positioned between the bubble manifold 202 and the instillation layer 2250. In some instances, the fluid distribution matrix 2252 may include additional tubing segments, as described above, which may be inserted between the bubble manifold 202 and the instillation layer 2250 at the time of manufacture, before the bubble manifold 202 and the instillation layer 2250 are attached or sealed together, for example by ultrasonic welding. In some embodiments, each of the fluid delivery conduits 2254 may be secured in place between the bubble manifold 202 and the instillation layer 2250 by welding the fenestrated manifold 1902, the bubble manifold 202, and the instillation layer 2250 together along at least some of the borders of the fluid delivery conduits 2254 and/or fluid channels 1940. In some embodiments, the fluid delivery conduits 2254 may have open ends, such as conduit ends 2360, and may also have fluid openings along the lengths of the fluid delivery conduits 2254 for delivering instillation fluid to the tissue site. In some alternative embodiments, the fluid delivery conduits 2254 may have only open ends, such as conduit ends 2360, and may otherwise be fluidly isolated along the lengths of the fluid delivery conduits 2254. In some additional embodiments, the fluid delivery conduits 2254 may have fluid openings along the lengths of the fluid delivery conduits 2254, but may have closed ends. Regardless of configuration of the fluid delivery conduits 2254, the tissue interface 108 of Figures 22 and 23 may be cut, sized, and customized to reduce the size of the tissue interface 108, which may therefore reduce the lengths of the fluid delivery conduits 2254.

Continuing with Figure 23, the fluid distribution hub 2256 may have a height that may allow the fluid distribution hub 2256 to extend from an outside surface of the tissue interface 108 through one or more layers of the tissue interface 108 and to be positioned in fluid communication with the fluid delivery conduits 2254. In some embodiments, the fluid distribution hub 2256 may extend outward from a surface of the instillation layer 2250 of the tissue interface 108, and at least a portion of the fluid distribution hub 2256 may be positioned between the bubble manifold 202 and the instillation layer 2250. The fluid distribution hub 2256 may include a first port on an upper surface, such as on the portion of the fluid distribution hub 2256 extending out from or above the instillation layer 2250, for receiving a fluid conduit, as well as a plurality of distribution ports positioned around the lower surface of the fluid distribution hub 2256. The distribution ports may be for fluidly coupling the fluid delivery conduits 2254 to the fluid distribution hub 2256. For example, the fluid delivery conduits 2254 may be positioned circumferentially about the fluid distribution hub 2256 and may extend radially away from the fluid distribution hub 2256 between the bubble manifold 202 and the instillation layer 2250. The fenestrated manifold 1902 of the tissue interface 108 may further include additional perforations through portions of the fenestrated manifold 1902 that may be aligned with the fluid delivery conduits 2254. The perforations may help facilitate the distribution of instillation fluids from the fluid delivery conduits 2254 to pass through the other layers of the tissue interface 108, such as the fenestrated manifold 1902, to a tissue site.

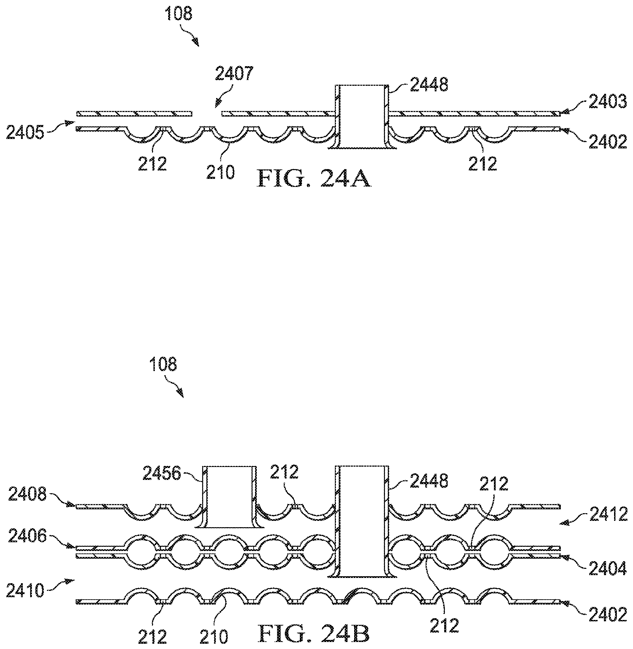

In some additional embodiments, a tissue interface that is suitable for both fluid removal and fluid instillation may include two or more layers of liquid-impermeable material, with at least one of the layers of liquid-impermeable material comprising bubbles. For example, as shown in Figure 24A, a tissue interface 108 suitable for both fluid removal and fluid instillation may include a first layer 2402 comprising bubbles 210 in the form of open-celled blisters, and a second liquid-impermeable layer 2403. The first layer 2402 may further include a plurality of apertures 212. The second liquid-impermeable layer 2403 may be positioned adjacent to the first layer 2402 and may be laminated or welded to the first layer 2402. In some embodiments, the welds joining the second liquid-impermeable layer 2403 to the first layer 2402 may be positioned so as to define one or more fluid channels 2405 in a space between the second liquid-impermeable layer 2403 and the first layer 2402. A fluid removal hub 2448 may extend through holes in both of the second liquid-impermeable layer 2403 and the first layer 2402 to facilitate delivery of negative pressure and fluid removal. Additionally, the second liquid-impermeable layer 2403 may include an aperture 2407 for allowing a fluid delivery conduit to provide instillation fluid to the one or more fluid channels 2405 between the second liquid-impermeable layer 2403 and the first layer 2402.

In some further embodiments, a tissue interface suitable for both fluid removal and fluid instillation may include two or more layers of liquid-impermeable material comprising bubbles. For example, the tissue interface 108 of Figure 2 may be doubled, tripled, or quadrupled, with the individual layers stacked on one another. Two instances of the tissue interface 108 of Figure 2 may be laminated or welded together to provide for a fluid delivery layer and a separate fluid removal layer. In such embodiments, one or both of the bubble layers may include a hole to allow communication with the other layer as well as an underlying tissue site. For example, a hole through a first, or top, layer could be included to allow for a fluid delivery conduit to provide instillation fluid to the space between the two layers. Welds joining the two layers may be placed so as to define fluid channels between the two layers through which fluid may be instilled. Another set of holes could be made through both of the first and second layers, where the holes in each of the layers are aligned to allow for a fluid removal conduit to deliver negative pressure to the tissue site beneath both bubble layers. Such holes could be punch-formed after manufacture. Although not required, an additional piece of manifolding material, such as a foam, could be positioned against a central portion of the top layer of the tissue interface, and in some embodiments, fluid delivery and/or fluid removal conduits could have open ends that terminate against a top surface of the foam, or alternatively one or more of the conduits could pass through the foam to the bubble layers below the foam.

For example, as shown in Figure 24B, a tissue interface 108 suitable for both fluid removal and fluid instillation may comprise four layers of liquid-impermeable material, with each of the four layers comprising bubbles. In some embodiments, the tissue interface 108 may include a first layer 2402, a second layer 2404, a third layer 2406, and a fourth layer 2408, and the four layers may be stacked on one another, with each of the four layers comprising bubbles 210 in the form of open-celled blisters. Each of the four liquid-impermeable layers may also include apertures 212 in order to allow fluid communication and manifolding through the layers. A first fluid pathway 2410 may be formed between the first layer 2402 and the second layer 2404 of the four layers, and may be suitable for communicating negative pressure. A second fluid pathway 2412 may be formed between the third layer 2406 and the fourth layer 2408, and may be suitable for delivering an instillation fluid. The bubbles 210 of each of the first layer 2402 and the second layer 2404 may protrude into the space between the first layer 2402 and the second layer 2404 defining the first fluid pathway 2410. Similarly, the bubbles 210 of each of the third layer 2406 and the fourth layer 2408 may protrude into the space between the third layer 2406 and the fourth layer 2408 defining the second fluid pathway 2412. A fluid removal hub 2448 may extend through the tissue interface 108 from a top surface of the tissue interface 108 to the first fluid pathway 2410 between the first layer 2402 and the second layer 2404 to facilitate delivery of negative pressure and fluid removal. A fluid distribution hub 2456 may extend from a top surface of the tissue interface 108 to the second fluid pathway 2412 between the third layer 2406 and the fourth layer 2408 to deliver instillation fluid. Portions of the four layers may be welded to each other, however at least portions of the peripheral edges of the four layers forming the tissue interface 108 may remain open so as to allow open pathways between the first fluid pathway 2410 and the second fluid pathway 2412 and the surrounding environment of a tissue site.

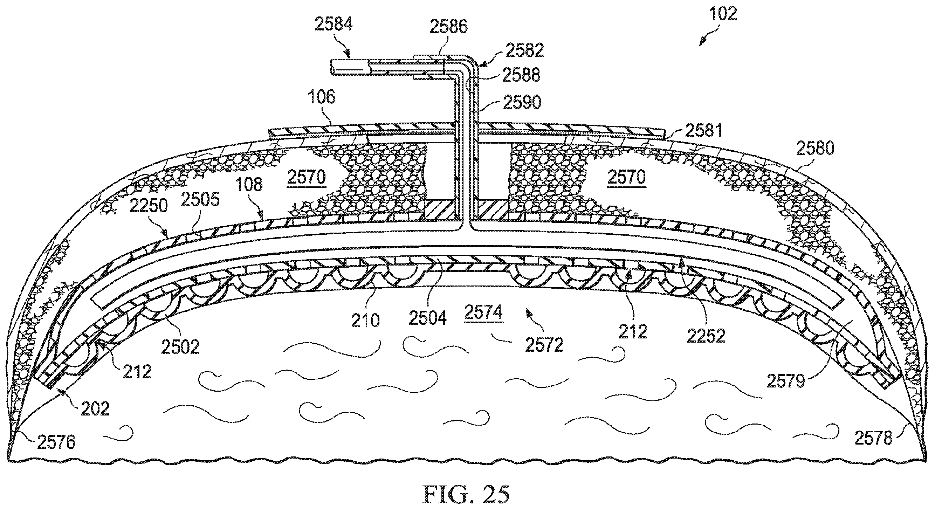

Figure 25 is a schematic diagram illustrating additional details that may be associated with some example embodiments of the therapy system 100. More specifically, Figure 25 shows a portion of an example embodiment of the dressing 102 for treating a tissue site 2572 within an abdominal cavity 2570. The tissue site 2572 may include the abdominal contents 2574 or tissue that is proximate the abdominal cavity 2570. Treatment of the tissue site 2572 may include removal of fluids, e.g., ascites, protection of the abdominal cavity 2570, or negative-pressure therapy.

As shown in Figure 25, a dressing 102 may include a tissue interface 108, which may be disposed within the abdominal cavity 2570 of a patient to treat the tissue site 2572. The tissue interface 108 may be supported by the abdominal contents 2574. As depicted, the tissue interface 108 may extend throughout a portion of the abdominal cavity 2570, so that a portion of the tissue interface 108 is positioned in or proximate to a first paracolic gutter 2576, and another portion of the tissue interface 108 is positioned in or proximate to a second paracolic gutter 2578. The first paracolic gutter 2576 and the second paracolic gutter 2578 may each be, for example, an open space on opposing sides of the abdominal cavity 2570, among the abdominal contents 2574. The first paracolic gutter 2576 may be laterally disposed from the second paracolic gutter 2578 or otherwise positioned on an opposite side of the tissue site 2572 from the second paracolic gutter 2578. Although Figure 25 depicts the dressing 102 deployed in the abdominal cavity 2570, the dressing 102 and therapy system 100 may be used at other types of tissue sites.

The example embodiment of the tissue interface 108 shown in Figure 25 may allow for the instillation of a treatment fluid in addition to the delivery of negative pressure, and accordingly, the tissue interface 108 is shown as comprising both a bubble manifold 202 and an instillation layer 2250. As shown in Figure 25, the bubble manifold 202 may include bubbles 210 comprising closed cells formed by two liquid-impermeable layers, the first layer 2502 and the second layer 25404. The instillation layer 2250 may also comprise a liquid-impermeable layer. The plurality of liquid-impermeable layers of the bubble manifold 202 of Figure 25, e.g., first layer 2502 and second layer 2504 may comprise apertures 212. Additionally, the instillation layer 2250 may have fenestrations 2505. The apertures 212 may take many shapes or combinations of shapes, including circular apertures or perforations, rectangular openings, fenestrations, or polygons, for example. The first layer 2502 may be adapted to be positioned between the second layer 2504 and the tissue site 2572 and abdominal contents 2574. The first layer 2502, second layer 2504, and the instillation layer 2250 may comprise a non-adherent material, such as a medical drape, capable of inhibiting tissue from adhering to the medical drape, such as a breathable polyurethane film. In the example embodiment of Figure 25, a chamber 2579 is formed between the second layer 2504 and the instillation layer 2250. In some embodiments, the chamber 2579 formed between the second layer 2504 and the instillation layer 2250 may include a fluid distribution matrix 2252 for delivering instillation fluid to the tissue site 2572.

The dressing 102 may further include a cover, such as cover 106, for providing a fluid seal over the tissue site 2572 and the abdominal cavity 2570. Additionally, one or more skin closure devices may be placed on an epidermis 2580 of a patient. An attachment device, such as attachment device 2581, may be used to attach the cover 106 to an attachment surface, such as the epidermis 2580 of the patient. In some embodiments, the dressing 102 may also include an interface 2582 for fluidly connecting the tissue interface 108 and other portions of the dressing 102 to a conduit 2584. The interface 2582 may include a connector 2586. Alternatively, the interface 2582 may be partially or fully embedded with a portion of the dressing 102, or configured in any other way possible for fluidly connecting the tissue interface 108 to a negative-pressure source 104 and/or solution source 114. The conduit 2584 may be fluidly coupled to the negative-pressure source 104 and/or solution source 114 of the therapy system 100 for providing negative pressure and/or treatment fluid, respectively, to the tissue interface 108. In some embodiments, the conduit 2584 may include two substantially parallel, fluidly-isolated conduits, one of which may be for fluidly coupling the tissue interface 108 to the negative-pressure source 104 and the other for fluidly coupling the tissue interface 108 to the solution source 114. Thus, in some embodiments, the conduit 2584 may be a multi-lumen conduit with both a negative-pressure lumen 2588 and a fluid supply lumen 2590. In other example embodiments, the conduit 2584 may be replaced with two separate conduits, one containing a negative-pressure lumen and the other containing a fluid supply lumen.

Although not necessarily depicted in Figure 25, in some embodiments, the dressing 102 may further include a filler material, such as a portion of a manifold material or foam, that is placed between the tissue interface 108 and the cover 106. The filler material may be sized to fill the portion of abdominal volume beneath or surrounding an incision or opening into the abdomen from the skin layers, such as a portion of the abdominal cavity 2570. In some embodiments, the filler material may serve as a distribution manifold for negative pressure. For example, in some embodiments, the filler material may be positioned between the tissue interface 108 and the cover 106, and a negative-pressure lumen or conduit, such as negative-pressure lumen 2588, may be pneumatically connected to the cover 106. As a result, fluid removal may occur from the layers of the dressing 102, including the tissue interface 108, through the filler material positioned atop the tissue interface 108, and into the negative-pressure lumen 2588. In some embodiments, the filler material may include an open-cell, reticulated polyurethane foam such as GRANUFOAM^{™} Dressing or VERAFLOW^{™} Therapy foam, both available from Kinetic Concepts, Inc. of San Antonio, Texas. The filler material may alternatively or additionally include a three-dimensional woven or non-woven fabric, such as TDL2 or TDL4, commercially available from Libeltex of Meulebeke, Belgium, or 3DXD or 4DXD spacer fabrics, commercially available from Baltex of Derbyshire, England.

In some embodiments, one or more components of the dressing 102 may additionally be treated with an antimicrobial agent. For example, the first layer 2502, the second layer 2504, or the instillation layer 2250 of the tissue interface 108 may be coated with an antimicrobial agent. In some embodiments, the first layer 2502, the second layer 2504, and/or the instillation layer 2250 may comprise a polymer coated or mixed with an antimicrobial agent. In other examples, the cover 106, the interface 2582, the conduit 2584, or other portion of the dressing 102 may additionally or alternatively be treated with one or more antimicrobial agents. Suitable antimicrobial agents may include, for example, metallic silver, PHMB, iodine or its complexes and mixes such as povidone iodine, copper metal compounds, chlorhexidine, or some combination of these materials. In some embodiments, the hydrophilicity of one or more layers of the disclosed tissue interfaces, such as the tissue interface 108, may be further enhanced with a coating, such as by a plasma coating process used by P2i Limited of Oxfordshire, United Kingdom, of another material to make the layer(s) more or less hydrophilic, or oleo-phobic.

In use, the tissue interface 108 may be sized to fit a given tissue site, such as tissue site 2572 within the abdominal cavity 2570, and disposed at or within the tissue site. For example, the tissue interface 108 may be cut to remove excess portions to appropriately size the tissue interface 108 to fit a compartment such as the abdominal cavity 2570. In some embodiments of the tissue interface 108, excess portions may be removed by cutting or tearing the liquid-impermeable layers, such as the first layer 2502, the second layer 2504, and the instillation layer 2250, altogether, optionally using some of the apertures or fenestrations of the layers of the tissue interface 108 as a guide.

Still referring primarily to Figure 25, the tissue interface 108 and other features of the dressing 102 may allow for the irrigation and washing out of an abdominal cavity, such as the abdominal cavity 2570, with the controlled and regulated introduction of fluid. In some instances, it may be necessary to wash or cleanse a contaminated abdominal cavity as a result of a perforated colon or sepsis. The therapy system 100 can provide means to instill fluid into an open abdomen to cleanse the abdominal contents, including reaching areas such as the small bowel loops, pancreas, etc. Additionally, the tissue interface 108 and other components of the dressing 102 and therapy system 100 may provide temporary closure to an open abdomen, while removing fluid and reducing edema. Thus, the therapy system 100 may provide the capability of performing washouts of a tissue site, such as abdominal cavity 2570, without having to repeatedly remove one or more dressings applied to the tissue site of a patient or brining the patient into the operating room for manual fluid introduction procedures. The therapy system 100 may thus be able to provide a controlled and regulated full abdominal wash, for example via instillation of a therapeutic fluid, as well as have the capability to provide a targeted wash to certain areas within the abdomen when required. Some embodiments of the therapy system 100, and more particularly the dressing 102, may also provide support and maintenance of the fascial domain of the abdominal cavity 2570, for example, and provide overall protection to the abdominal contents 2574.

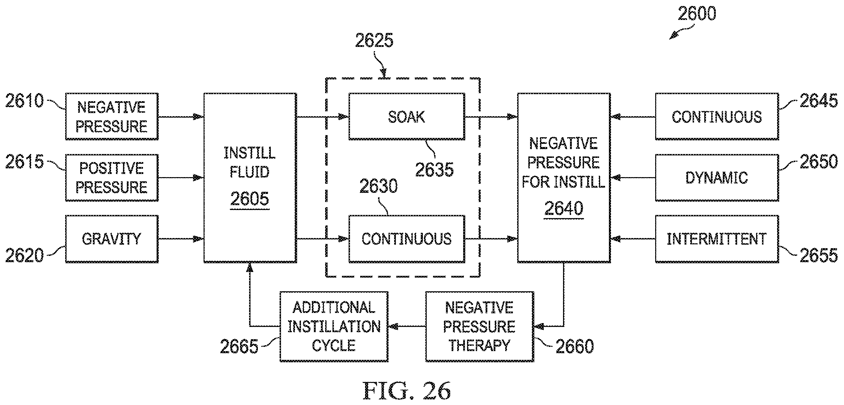

Figure 26 is a chart illustrating details that may be associated with an example method 2600 of operating the therapy system 100 to provide negative-pressure treatment and instillation treatment to the tissue interface 108. In some embodiments, the controller 110 may receive and process data, such as data related to instillation solution provided to the tissue interface 108. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second and 30 minutes. The controller 110 may also control the operation of one or more components of the therapy system 100 to instill solution, as indicated at 2605. For example, the controller 110 may manage fluid distributed from the solution source 114 to the tissue interface 108. In some embodiments, fluid may be instilled to a tissue site by applying a negative pressure from the negative-pressure source 104 to reduce the pressure at the tissue site, drawing solution into the tissue interface 108, as indicated at 2610. In some embodiments, solution may be instilled to a tissue site by applying a positive pressure from the positive-pressure source 116 to move solution from the solution source 114 to the tissue interface 108, as indicated at 2615. Additionally or alternatively, the solution source 114 may be elevated to a height sufficient to allow gravity to move solution into the tissue interface 108, as indicated at 2620.

The controller 110 may also control the fluid dynamics of instillation at 2625 by providing a continuous flow of solution at 2630 or an intermittent flow of solution at 2635. Negative pressure may be applied to provide either continuous flow or intermittent flow of solution at 2640. The application of negative pressure may be implemented to provide a continuous pressure mode of operation at 2645 to achieve a continuous flow rate of instillation solution through the tissue interface 108, or it may be implemented to provide a dynamic pressure mode of operation at 2650 to vary the flow rate of instillation solution through the tissue interface 108. Alternatively, the application of negative pressure may be implemented to provide an intermittent mode of operation at 2655 to allow instillation solution to dwell at the tissue interface 108. In an intermittent mode, a specific fill volume and dwell time may be provided depending, for example, on the type of tissue site being treated and the type of dressing being utilized. After or during instillation of solution, negative-pressure treatment may be applied at 2660. The controller 110 may be utilized to select a mode of operation and the duration of the negative pressure treatment before commencing another instillation cycle at 2665 by instilling more solution at 2605.

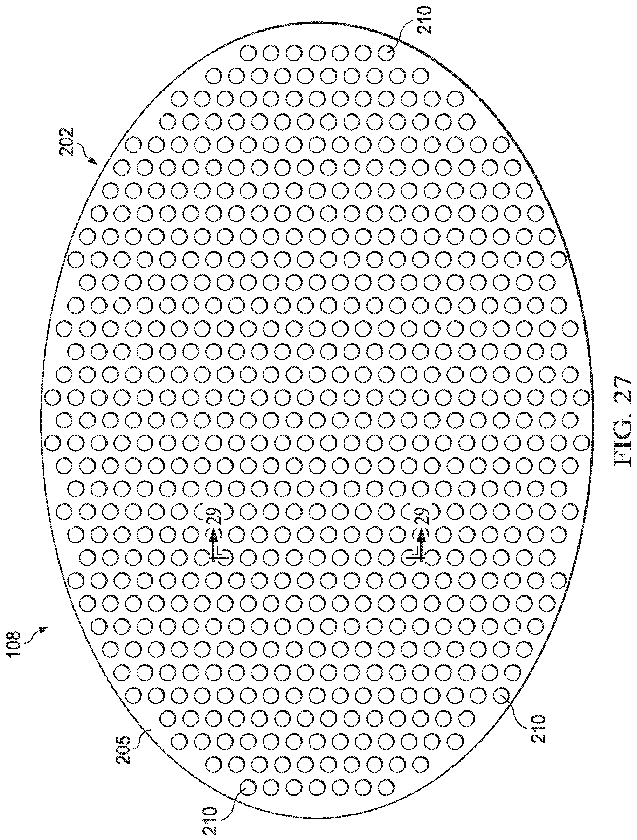

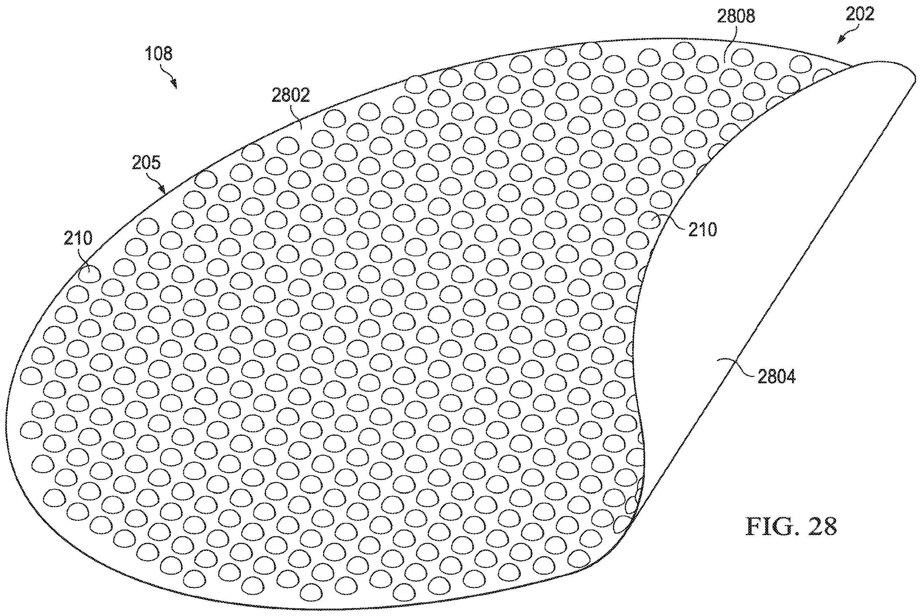

Figures 27 and 28 show another tissue interface 108 that may be associated with some embodiments of the therapy system 100 of Figure 1, illustrating additional details that may be associated with some embodiments. The tissue interface 108 may comprise or consist essentially of a bubble manifold 202 having a liquid-impermeable layer 205 with a first surface 2802, a second surface 2804, and a thickness separating the first surface 2802 and the second surface 2804; and a plurality of bubbles 210 disposed on at least one surface (e.g. the first surface 2802). In some embodiments, the bubbles 210 may extend outward from at least the first surface 2802, which may be an outer surface of the liquid-impermeable layer 205. In some embodiments, the bubbles 210 may each have a height of approximately 1.0 mm - 10.0 mm, approximately 3.0 mm - 6.0 mm, or approximately 9.0 - 10.0 mm. In some embodiments, the bubbles 210 may have a diameter or width of approximately 3.0 mm - 12.0 mm, approximately 3.0 - 5.0 mm, approximately 6.0 mm - 12.0 mm, or approximately 12 mm. In some embodiments, each of the bubbles may be approximately hemispherical or dome-like in shape (e.g. half-spheres). In some embodiments, the plurality of bubbles 210 may be substantially coextensive with at least one surface of the liquid-impermeable layer 205 (e.g. the first surface 2802). In some embodiments, the plurality of bubbles 210 may all be uniformly spaced on at least one surface of the liquid-impermeable layer 205 (e.g. the first surface 2802). For example, spacing of the plurality of bubbles 210 may form gaps 2808, which may be approximately 1.0-6.0 mm, approximately 1.0-3.0 mm, or approximately 3.0-6.0 mm between adjacent bubbles 210. In this context, uniform may mean exactly uniform or substantially uniform (e.g. not absolutely uniform, and including variations which are approximately uniform or within acceptable manufacturing tolerances). In some embodiments, the bubbles 210 may be arranged in a staggered pattern (e.g. in offset rows) on at least the first surface 2802, for example so as not to be aligned in a grid-like pattern. In some embodiments, both the first surface 2802 and the second surface 2804 may be configured to contact the tissue site within abdominal cavity. In some embodiments, the tissue interface 108 may be elongate, for example having a length which is greater than its width. In some embodiments, the width may be approximately 80-85% of the length in size. For example, the tissue interface 108 may have a length of approximately 802 mm and a width of approximately 665 mm. In some embodiments, the tissue interface 108 may have an oval shape.

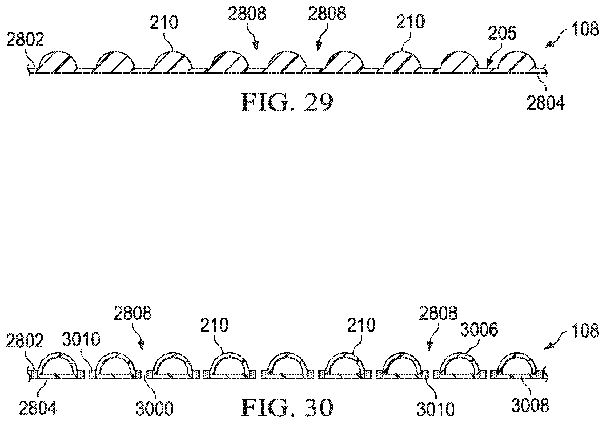

Figure 29 is a section view of an example embodiment cross-section of Figure 28, illustrating additional details that may be associated with some embodiments. In some embodiments, each of the plurality of bubbles 210 may be solid. In some embodiments, the liquid-impermeable layer 205 may comprise a single polymer film layer having solid bubbles 210 extending from at least one outer surface, for example with the bubbles 210 extending from the first surface 2802. In some embodiments, the plurality of bubbles 210 may each be formed of the same polymer material as the film layer. Although not shown, some embodiments may comprise fenestrations (such as apertures 212), for example located between bubbles 210 in the gaps 2808.

Figure 30 is a section view of another example embodiment cross-section of Figure 28, illustrating additional details that may be associated with some embodiments. In some embodiments, the liquid-impermeable layer 205 may further comprise a plurality of fenestrations 3000 (e.g. similar to apertures 212), for example positioned between the bubbles 210. The fenestrations 3000 may be configured to allow fluid flow through the liquid-impermeable layer 205, for example forming apertures that pass entirely through the thickness of the liquid-impermeable layer 205 (e.g. between the first surface 2802 and the second surface 2804). In some embodiments, the liquid-impermeable layer 205 may comprise a first sheet 3006 of polymeric film (e.g. similar to first layer 502) and a second sheet 3008 of polymeric film (e.g. similar to second layer 504). For example, inner surfaces of each of the first sheet 3006 of polymeric film and the second sheet 3008 of polymeric film may be sealed to each other to form an integral liquid-impermeable layer 205. In some embodiments, there may be nothing interposed between the first sheet 3006 and the second sheet 3008. For example, there may be no foam disposed between the two sheets of polymeric film. In some embodiments, the first sheet 3006 may directly contact the second sheet 3008. The outer surface of the first sheet 3006 may form the first surface 2802 of the liquid-impermeable layer 205, and the outer surface of the second sheet 3008 may form the second surface 2804 of the liquid-impermeable layer 205.

In some embodiments, attachment of the first sheet 3006 and the second sheet 3008 may form enclosures between the inner surfaces of the sheets that define the bubbles 210. In some embodiments, the first sheet 3006 may comprise the plurality of bubbles 210 (e.g. indentations projecting outward from the outer surface of the first sheet 3006), and the second sheet 3008 may be substantially flat or free of bubbles. In some embodiments, each of the plurality of bubbles 210 may comprise a hollow closed-cell (e.g. closed so there is no opening to the bubble cavity or interior of the hollow bubble). For example, the first sheet 3006 and the second sheet 3008 may be sealed together around each bubble, so that each of the plurality of bubbles 210 may be individually sealed as a closed-cell. In some embodiments, each hollow closed-cell may comprise gas (e.g. air) within. In some embodiments, the gas within each closed-cell bubble may be at approximately atmospheric pressure. While some embodiments may have hollow bubbles 210, other embodiments, may have solid bubbles. Solid bubbles may provide more support in resisting contraction to maintain an open flowpath, and hollow bubbles may provide more comfort. In some embodiments, the two sheets of polymeric film 3006 and 3008 may have inner surfaces attached only in the gaps 2808 between the plurality of bubbles 210. For example, the inner surfaces may be attached by welds 3010, such as heat welds, RF welds, or ultrasonic welds, and the welds 3010 may be located entirely within the gaps 2808 between the plurality of bubbles 210. In some embodiments, the welds 3010 may span the entire gap 2808 between adjacent bubbles 210. In some embodiments, the fenestrations 3000 may extend through the welds 3010.

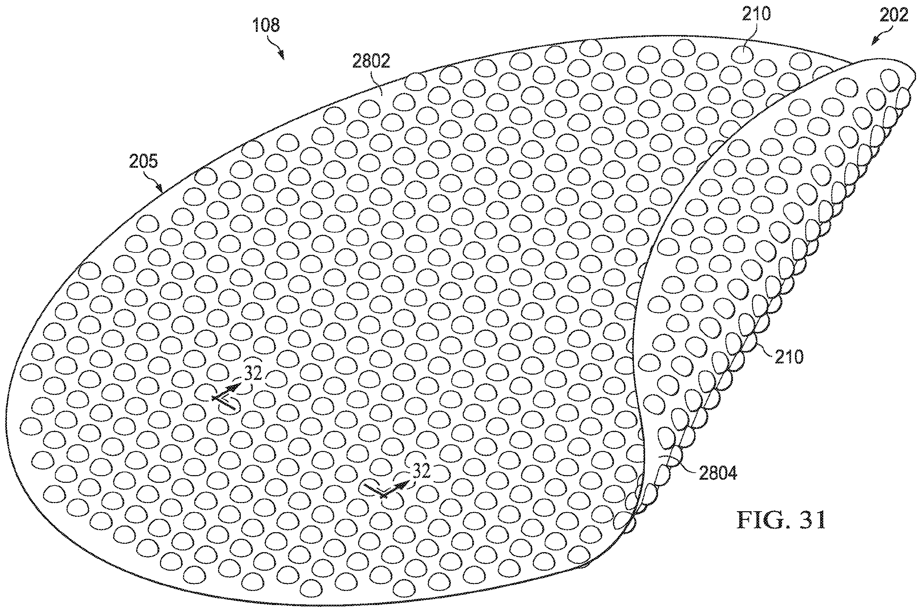

Figure 31 is an isometric view of an illustrative embodiment of yet another tissue interface 108 that may be associated with some embodiments of the therapy system 100 of Figure 1, illustrating additional details that may be associated with some embodiments. The tissue interface 108 (e.g. bubble manifold 202) of Figure 31 may be similar to that of Figure 27, and may additionally have bubbles 210 on both surfaces. For example, the liquid-impermeable layer 205 may further comprise the second surface 2804 opposite the first surface 2802 (e.g. separated by a thickness of the liquid-impermeable layer 205), and the plurality of bubbles 210 may extend outward from both the first surface 2802 and the second surface 2804. In some embodiments, the plurality of bubbles 210 may be disposed on both the first surface 2802 and the second surface 2804, extending outward from the liquid-impermeable layer 205. In some embodiments, the plurality of bubbles 210 may be substantially coextensive with the first surface 2802 and the second surface 2804. In some embodiments, the plurality of bubbles 210 may all be uniformly spaced on the first surface 2802 and the second surface 2804. In some embodiments, the bubbles 210 on the first surface 2802 may be substantially coextensive with the first surface 2802, the bubbles 210 on the second surface 2804 may be substantially coextensive with the second surface 2804, the bubbles 210 on the first surface 2802 may be uniformly spaced on the first surface 2802, and/or the bubbles 210 on the second surface 2804 may be uniformly spaced on the second surface 2804. In some embodiments, the bubbles 210 may be configured to provide an average fluid flow rate of at least 600 mL/min, for example approximately 647.78 mL/min, under negative-pressure therapy.

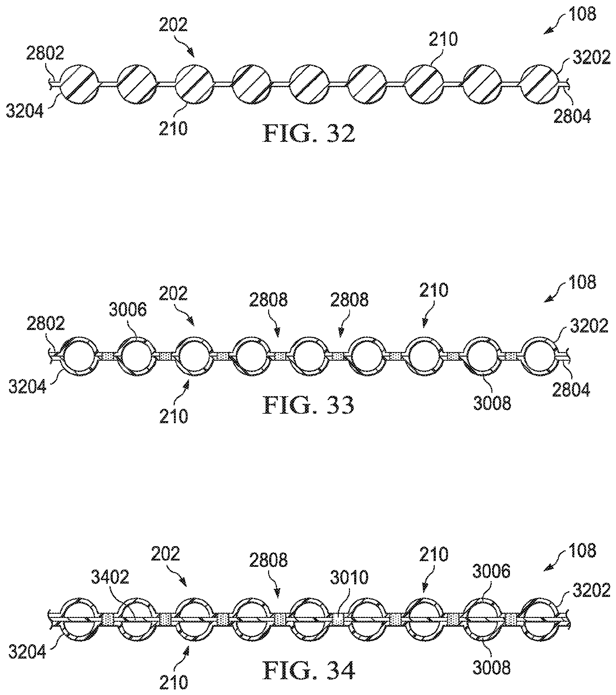

Figure 32 is a section view of an example embodiment cross-section of Figure 31, illustrating additional details that may be associated with some embodiments. Some embodiments may be similar to those described with respect to Figure 29, but the plurality of bubbles 210 may also extend outward from the second surface 2804 of the liquid-impermeable layer 205. In some embodiments, the plurality of bubbles 210 may extend from both the first surface 2802 and the second surface 2804 (e.g. both outer surfaces) of a single polymer film layer. For example, the plurality of bubbles 210 may comprise a first set 3202 of bubbles 210 extending outward from the first surface 2802 and a second set 3204 of bubbles 210 extending outward from the second surface 2804. In some embodiments, solid bubbles 210 may extend outward from both outer surfaces. In some embodiments, the first set 3202 of bubbles 210 and the second set 3204 of bubbles 210 may be aligned or stacked, as shown in Figure 32, but in other embodiments the first set 3202 of bubbles 210 may be offset or non-aligned with the second set 3204 of bubbles 210. In some embodiments with aligned bubbles 210, the aligned bubbles 210 may jointly form spherical or ovoidal supports. Although not shown, some embodiments may comprise fenestrations, for example located between bubbles 210.

Figure 33 is a section view of another example embodiment cross-section of Figure 31, illustrating additional details that may be associated with some embodiments. The liquid-impermeable layer 205 of Figure 33 may comprise a first sheet 3006 of polymeric film and a second sheet 3008 of polymeric film. Some embodiments may be similar to those described with respect to Figure 30, but the plurality of bubbles 210 may also extend outward from the outer surface of the second sheet 3008 of polymeric film. For example, the first sheet 3006 may comprise the first set 3202 of bubbles 210 extending outward from its outer surface (e.g. the first surface 2802 of the liquid-impermeable layer 205), and the second sheet 3008 may comprise the second set 3204 of bubbles 210 extending outward from its outer surface (e.g. the second surface 2804 of the liquid-impermeable layer 205). In some embodiments, the first set 3202 of bubbles 210 and the second set 3204 of bubbles 210 may each be hollow. In some embodiments, the first sheet 3006 and the second sheet 3008 may be sealed together, for example with inner surfaces sealed to form sealed hollow bubbles 210. For example, the first sheet 3006 and the second sheet 3008 may be welded together in the gap 2808 between bubbles 210 to form an integral liquid-impermeable layer 205 with external bubbles 210 on the outer-facing surfaces. In some embodiments, the first set 3202 of bubbles 210 and the second set 3204 of bubbles 210 may align or be stacked, to form larger support bubbles 210 extending from both surfaces, as shown in Figure 33, but in other embodiments the first set 3202 of bubbles 210 may be offset or non-aligned with the second set 3204 of bubbles 210. Although not shown, some embodiments may comprise fenestrations, for example located between bubbles 210.

Figure 34 is a section view of yet another example embodiment cross-section of Figure 31, illustrating additional details that may be associated with some embodiments. The bubble manifold 202 of Figure 34 may be similar to Figure 33, but may further comprise a third sheet 3402 of polymeric film (e.g. similar to third layer 820), which may be disposed between and sealed to both the first sheet 3006 and the second sheet 3008. In some embodiments, the third sheet 3402 may be formed of the same material as the first sheet 3006 and the second sheet 3008. For example, all the sheets may be formed of the same polymeric material, such as polyurethane film. In some embodiments, the first sheet 3006 may have the first set 3202 of bubbles 210 extending outward from its outer surface, and the second sheet 3008 may have the second set 3204 of bubbles 210 extending outward from its outer surface. The inner surface of the first sheet 3006 may be attached to the third sheet 3402, and the inner surface of the second sheet 3008 may be attached to the third sheet 3402, for example opposite the first sheet 3006. In some embodiments, the third sheet 3402 may be substantially flat or free of bubbles 210. In some embodiments, the first sheet 3006 may be sealed to the third sheet 3402, and the second sheet 3008 may be sealed to the third sheet 3402 (e.g. on opposite sides of the third sheet 3402). In some embodiments, the first sheet 3006 and the second sheet 3008 may be only indirectly sealed together, for example via sealing to the interposing third sheet 3402.

In some embodiments, the first set 3202 of bubbles 210 and the second set 3204 of bubbles 210 may be hollow. In some embodiments, the first set 3202 of bubbles 210 and the second set 3204 of bubbles 210 may be aligned and/or stacked, as shown in Figure 34, but in other embodiments the first set 3202 of bubbles 210 and the second set 3204 of bubbles 210 may be offset or non-aligned. In some embodiments, even when the first set 3202 of bubbles 210 and the second set 3204 of bubbles 210 are aligned, there may still be no fluid communication therebetween (e.g. between the aligned hollow bubble cavities). For example, the third sheet 3402 of polymeric film may divide and fluidly separate the first set 3202 of bubbles 210 from the second set 3204 of bubbles 210. In other embodiments, the third sheet 3402 may be perforated or porous to permit airflow between the aligned hollow bubble cavities. In some embodiments, the first sheet 3006, second sheet 3008, and third sheet 3402 may be attached in the gaps 2808 between bubbles 210. For example, all the gaps 2808 between bubbles 210 may comprise a weld 3010, which may attach and the sheets and seal the bubbles 210. Although not shown, some embodiments may comprise fenestrations, for example located between bubbles 210.

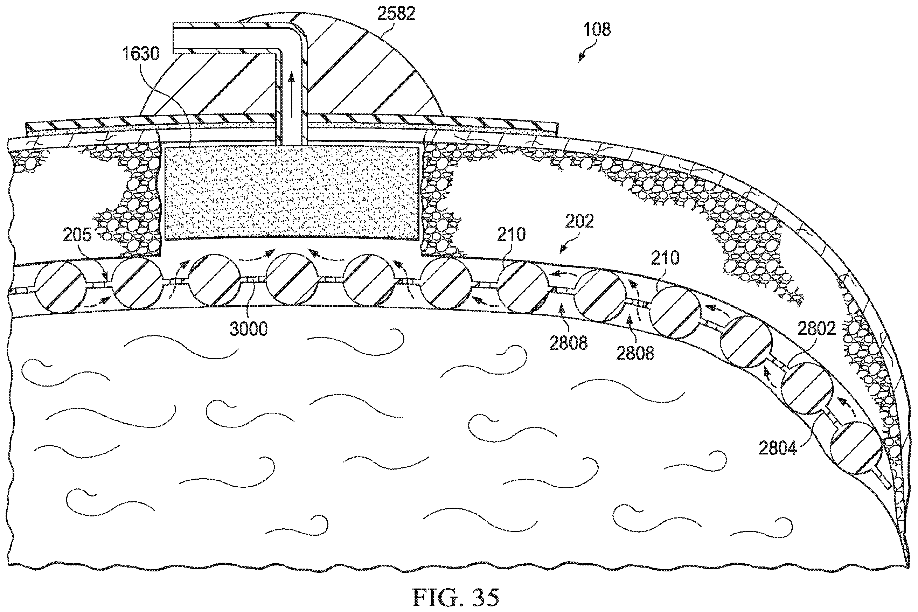

Figure 35 is a schematic diagram, with a portion in cross-section, of an illustrative dressing for treating a tissue site, which may comprise a tissue interface 108 (e.g. bubble manifold 202) similar to that of Figure 31 and may be associated with some embodiments of the therapy system 100 of Figure 1. In some embodiments, the gap 2808 spacing between the plurality of bubbles 210 may form an external fluid pathway (e.g. along one or more outer surfaces of the liquid-impermeable layer 205 between the bubbles 210), which may be configured to distribute negative pressure across the tissue interface 108 during negative-pressure therapy, to allow for liquid (e.g. exudate) removal when under negative pressure, and/or to distribute instillation therapy. Optional fenestrations 3000 may improve fluid movement by allowing fluid movement between the first surface 2802 and second surface 2804. In some embodiments, a foam manifold 1630 may be placed atop the bubble manifold 202, and may be configured to distribute negative pressure to the bubble manifold 202. In some embodiments, the foam manifold 1630 may be disposed between the bubble manifold 202 and the dressing interface 2582.

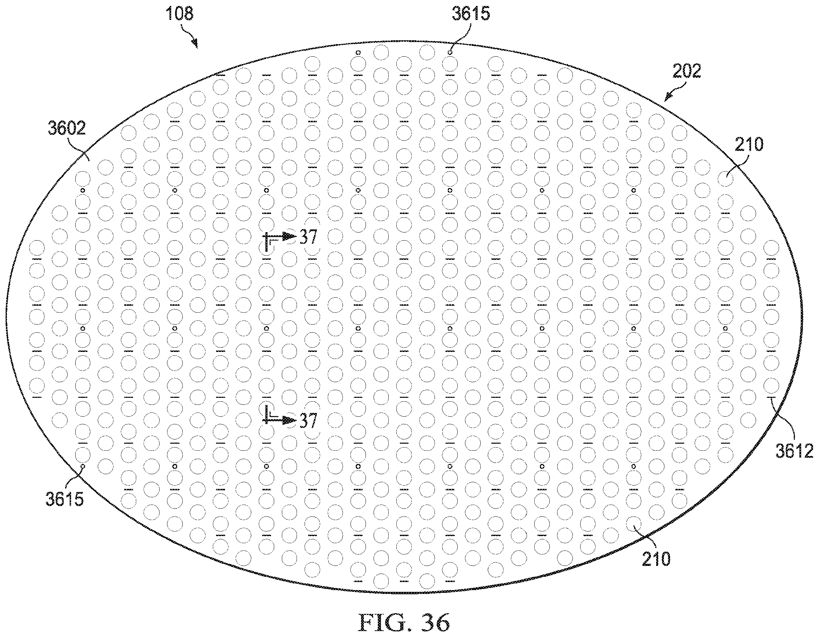

Figure 36 is a schematic, plan view of an illustrative embodiment of still another tissue interface 108 that may be associated with some embodiments of the therapy system 100 of Figure 1, illustrating additional details that may be associated with some embodiments. In some embodiments, a bubble manifold 202 similar to that of Figures 27-34 may be encapsulated with film to form one or more internal fluid pathway 3610. In addition to a bubble manifold 202 (e.g. a liquid-impermeable layer 205 having external bubbles 210 located on one or both outer surfaces), the embodiment of Figure 36 may comprise at least one encapsulating film 3602 attached to the liquid-impermeable layer 205 and/or bubbles 210 to cover the bubbles 210 of the liquid-impermeable layer 205, which may form an internal fluid pathway 3610 between the liquid-impermeable layer 205 and the encapsulating film 3602. For example, the bubbles 210 may support the encapsulating film 3602 over the surface of the liquid-impermeable layer 205 in gap 2808 locations. The encapsulating film 3602 may be fenestrated, for example with fenestrations 3612 configured to allow fluid communication between the internal fluid pathway 3610 and the tissue site external to the encapsulating film 3602. In some embodiments, the encapsulating film 3602 may be configured to contact the tissue site. In some embodiments, the encapsulating film 3602 may be attached to one or more outer surface of the liquid-impermeable layer 205. For example, the encapsulating film 3602 may be welded to the liquid-impermeable layer 205 in the gaps 2808 between bubbles 210. In some embodiments, the welds may be spot welds 3615, which may be spaced apart approximately 2-3 inches. Some embodiments may be configured to provide an average fluid flow rate of approximately 24 mL/min.

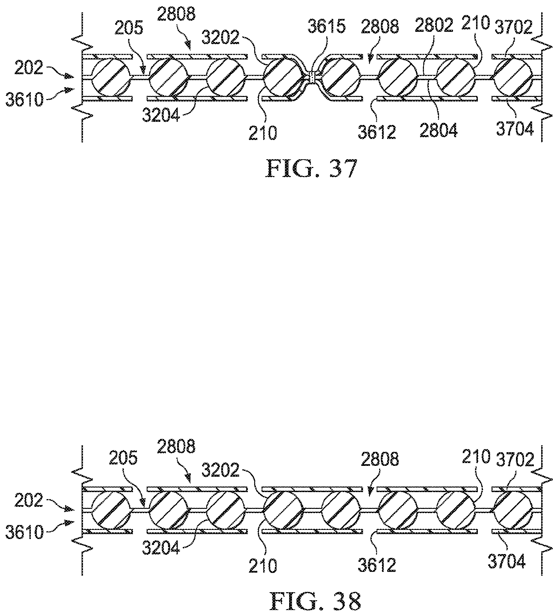

Figure 37 is a section view of an example embodiment cross-section of Figure 36, illustrating additional details that may be associated with some embodiments. Some embodiments may have bubbles 210 extending from both the first surface 2802 and second surface 2804 of the liquid-impermeable layer 205 of the bubble manifold 202. For example, the first set 3202 of bubbles 210 may extend from the first surface 2802, and the second set 3204 of bubbles 210 may extend form the second surface 2804. In some embodiments, the at least one encapsulating film 3602 may comprise a first encapsulating film 3702 and a second encapsulating film 3704. In some embodiments, the first encapsulating film 3702 may contact the first set 3202 of bubbles 210 (e.g. which extend from the first surface 2802 of the liquid-impermeable layer 205), and the second encapsulating film 3704 may contact the second set 3204 of bubbles 210 (e.g. which extend from the second surface 2804 of the liquid-impermeable layer 205). In some embodiments, the plurality of bubbles 210 may be configured to support the at least one encapsulating layer 3602 above the gaps 2808 between bubbles 210, for example maintaining one or more open fluid pathway during negative-pressure therapy. For example, the first set 3202 of bubbles 210 may support the first encapsulating film 3702, and the second set 3204 of bubbles 210 may support the second encapsulating film 3704.

In some embodiments, the first encapsulating film 3702 may be attached (e.g. coupled) to the first surface 2802 of the liquid-impermeable layer 205, and the second encapsulating film 3704 may be attached to the second surface 2804 of the liquid-impermeable layer 205. For example, the attachment of the first surface 2802 to the first encapsulating film 3702 and the second surface 2804 to the second encapsulating film 3704 may comprise welds. In some embodiments, the welds may be located entirely within the gaps 2808 between adjacent bubbles 210. In some embodiments, the welds 3010 may be spot welds 3615. In some embodiments, the first encapsulating layer 3702 and the second encapsulating layer 3704 may also be sealingly attached (e.g. welded) to each other at a perimeter. In some embodiments, the first encapsulating film 3702 and/or the second encapsulating film 3704 may be fenestrated, for example with fenestrations 3612 disposed over the gaps 2808 between the bubbles 210.

Figure 38 is a section view of another example embodiment cross-section of Figure 36, illustrating additional details that may be associated with some embodiments. In some embodiments, the bubble manifold 202 of Figure 38 may be similar to Figure 37, except that the at least one encapsulating film 3602 may be attached, for example by adhesive, atop the bubbles 210 (e.g. attached to distal ends of the bubbles 210, with no attachment of the at least one encapsulating film 3602 to the liquid-impermeable layer 205 in the gaps 2808 between bubbles 210). For example, the first encapsulating film 3702 may be attached to the first set 3202 of bubbles 210, and the second encapsulating film 3704 may be attached to the second set 3204 of bubbles 210. In some embodiments, the encapsulating layers may be attached to the crown or distal end (e.g. atop) of the bubbles 210. In some embodiments, there may be no attachment of encapsulating films to the liquid-impermeable layer 205 between bubbles 210. In some embodiments, the first encapsulating film 3702 and/or second encapsulating film 3704 may be fenestrated, for example with the fenestrations 3612 disposed over the gaps 2808 between bubbles 210.

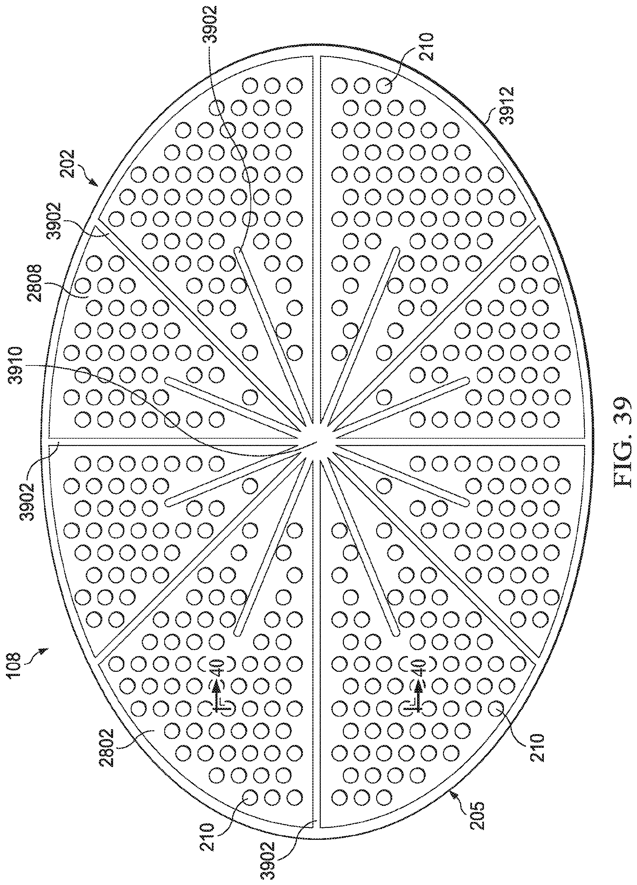

Figure 39 is a schematic, plan view of an illustrative embodiment of still another tissue interface 108 that may be associated with some embodiments of the therapy system 100 of Figure 1, illustrating additional details that may be associated with some embodiments. The bubble manifold 202 of Figure 39 may be similar to Figure 27 or Figure 31, and may further comprise one or more break-lines 3902. For example, some tissue interface 108 embodiments may comprise at least one liquid-impermeable layer 205; a plurality of bubbles 210 disposed on at least a first surface 2802 of the liquid-impermeable layer 205; and a pattern of break-lines 3902 configured to separate the plurality of bubbles 210 into a plurality of zones on at least the first surface 2802. In some embodiments, the perimeter 3912 of the liquid-impermeable layer 205 may be sealed, for example with a weld.

In some embodiments, each break-line may be free of bubbles. In some embodiments, the break-lines 3902 may each comprise portions of the first surface 2802 and/or second surface 2804 of the liquid-impermeable layer 205 between bubbles 210 that are free of bubbles (e.g. substantially flat). In some embodiments, the break-lines 3902 may each comprise bubbles that have been permanently compressed to substantially flat, for example by welding of the bubbles. For example, the break-lines 3902 may be formed by welding the liquid-impermeable layer 205 to form a break between zones that is larger than the gap 2808 between bubbles 210. In some embodiments, each break-line 3902 may be wider than the gap 2808 between adjacent bubbles 210 (e.g. in zones adjacent the break-line). In some embodiments, each break-line 3902 may be at least twice as wide as the gap 2808 between adjacent bubbles 210. In some embodiments, each break-line 3902 may have a width approximately equal to the width of a bubble 210 plus the width of two gaps 2808. Each break-line 3902 has a width of approximately 3 0-24.0 mm, approximately 12.0-18.0 mm, and/or approximately 18.0-24.0 mm. In some embodiments, each break-line 3902 may be configured to restrict fluid flow across its width more than the gap 2808 between bubbles 210. In some embodiments, the bubbles 210 within each zone may be substantially uniformly spaced, forming the gap 2808 between adjacent bubbles 210. In some embodiments, the pattern of break-lines 3902 may be disposed on both the first surface 2802 and the second surface 2804 (e.g. both outer surfaces) of the liquid-impermeable layer 205. In some embodiments, the pattern of break-lines 3902 may be configured to define one or more surface flow channels configured to allow a flow rate of approximately 166-481 mL/min or approximately 208-481 mL/min under negative-pressure therapy. In some embodiments, the pattern of break-lines may be configured to allow a flow rate during negative-pressure therapy of greater than 4.5 mL/min, greater than 24 mL/min, greater than 166 mL/min, less than 650 mL/min, approximately 24-647 mL/min, approximately 24-500 mL/min, approximately 150-500 mL/min, and/or approximately 150-600 mL/min.

In some embodiments, the pattern of break-lines 3902 may be configured so that no break-lines 3902 are substantially perpendicular to a radius of the liquid-impermeable layer 205. In some embodiments, the break-lines 3902 may not cross or otherwise impeded radial flow across the liquid-impermeable layer 205 (e.g. in the gaps between bubbles). One or more of the break-lines 3902 are straight. For example, in Figure 39, all of the break-lines 3902 are straight lines. At least some of the break-lines 3902 extend radially. For example, in Figure 39, all of the break-lines 3902 may extend radially from a center 3910 of the liquid-impermeable layer 205. In some embodiments, some or all of the radially-extending break-lines 3902 may extend from substantially the center 3910 to substantially a perimeter 3912 or periphery of the liquid-impermeable layer 205. For example, as used herein, the radius of the liquid-impermeable layer 205 may extend from the center 3910 to the perimeter 3912, regardless of the shape of the liquid-impermeable layer 205. In some embodiments, the break-lines 3902 extending from substantially the center 3910 of the liquid-impermeable layer 205 to substantially a perimeter 3912 of the liquid-impermeable layer 205 (e.g. full break-lines) may be spaced approximately 45 degrees apart. In some embodiments, some of the break-lines 3902 (e.g. partial break-lines) may additionally extend at least half a distance from substantially the center 3910 to substantially the perimeter 3912 (e.g. extending from the center 3910 to substantially a midpoint of a radius), and may be located substantially half way between the arc of adjacent break-lines 3902 extending from substantially a center 3910 of the liquid-impermeable layer 205 to substantially a perimeter 3912 of the liquid-impermeable layer 205 (e.g. bisecting the arc between full-length radius break-lines). In some embodiments, the break-line pattern shown in Figure 39 may be a star pattern, and may have an average fluid removal rate of approximately 208.67 mL/min under negative-pressure therapy.

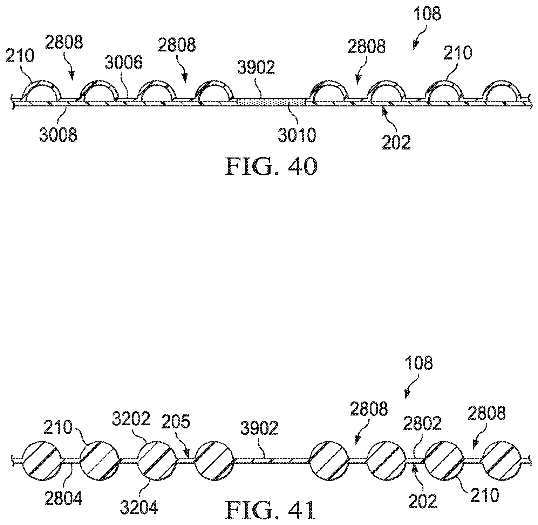

Figure 40 is a section view of an example embodiment cross-section of Figure 39, illustrating additional details that may be associated with some embodiments. Figure 40 illustrates an embodiment having bubbles 210 and the pattern of break-lines 3902 located on only one surface (e.g. the first surface 2802 of the liquid-impermeable layer 205). In some embodiments, the bubble manifold 202 may be similar to that of Figure 30, with the addition of one or more break-lines 3902 forming or dividing zones of bubbles 210. In some embodiments, the liquid-impermeable layer 205 may comprise the first sheet 3006 of polymeric film and the second sheet 3008 of polymeric film. For example, the tissue interface 108 may comprise: the first sheet 3006 of polymeric film having an inner surface, an outer surface, and a thickness extending between the inner surface and the outer surface; and the second sheet 3008 of polymeric film having an inner surface, an outer surface, and a thickness extending between the inner surface and the outer surface, wherein the inner surface of the first sheet 3006 of polymeric film and the inner surface of the second sheet 3008 of polymeric film are sealingly attached. In some embodiments, the plurality of bubbles 210 may extend outward from the outer surface of at least the first sheet 3006 of polymeric film, and the pattern of break-lines 3902 may be configured to separate the plurality of bubbles 210 into the plurality of zones on the outer surface of at least the first sheet 3006 of polymeric film.

In some embodiments, each break-line may be free of bubbles and may be wider than the gap 2808 between adjacent bubbles 210. For example, each break-line 3902 may be at least twice as wide as the gap 2808. In some embodiments, the first sheet 3006 of polymeric film and the second sheet 3008 of polymeric film may be sealed to form the plurality of bubbles 210 without open bubble cavities (e.g. with enclosed, hollow bubble cavities). In some embodiments, the bubbles 210 may extend from the outer surface of the first sheet 3006 of polymeric film. In some embodiments, there may be no bubbles extending from the outer surface of the second sheet 3008 of polymeric film. In some embodiments, the break-lines 3902 may comprise welds 3010 attaching the first sheet 3006 of polymeric film to the second sheet 3008 of polymeric film. For example, the inner surfaces of the first sheet 3006 and the second sheet 3008 may be attached at the break-lines 3902. In some embodiments, the inner surfaces of the first sheet 3006 and the second sheet 3008 may substantially only be attached at the break-lines 3902 and/or the perimeter. While Figure 40 illustrates the bubbles 210 as hollow, in other embodiments, the bubbles 210 may be solid (e.g. with solid bubbles 210 extending from a surface of a single polymer film). Although not shown here, some embodiments may further comprise fenestrations, which may be located between bubbles 210. In some embodiments, the fenestrations may be located in the gaps 2808 and/or in the break-lines 3902.

Figure 41 is a section view of another example embodiment cross-section of Figure 39, illustrating additional details that may be associated with some embodiments. In some embodiments, the bubble manifold 202 of Figure 41 may be similar to that of Figure 32, and further may include break-lines 3902. In some embodiments, the bubble manifold 202 of Figure 41 may be similar to that of Figure 40, and may have bubbles 210 extending from both the first surface 2802 and the second surface 2804 (e.g. the first set 3202 of bubbles 210 extending from the first surface 2802, and the second set 3204 of bubbles 210 extending from the second surface 2804). In some embodiments, the pattern of break-lines 3902 may be formed on both the first surface 2802 and the second surface 2804 of the liquid-impermeable layer 205. For example, the pattern of break-lines 3902 may also separate the plurality of bubbles 210 into a plurality of zones on the outer surface of the second sheet 3008 of polymeric film (e.g. break-lines 3902 may be disposed on both the first surface 2802 and second surface 2804). In some embodiments, the break-lines 3902 may separate the first set 3202 of bubbles 210 and the second set 3204 of bubbles 210 into zones.

Typically, the break-lines 3902 may be wider than the gap 2808 between bubbles 210 in the zones. For example, the break-lines 3902 may each be at least twice as wide as the gap 2808 between bubbles 210. While Figure 41 illustrates the first set 3202 of bubbles 210 aligned and stacked with the second set 3204 of bubbles 210 (e.g. mirror images across the liquid-impermeable layer 205), in other embodiments the first set 3202 of bubbles 210 and the second set 3204 of bubbles 210 may not be aligned (e.g. may be offset). While Figure 41 illustrates the break-lines 3902 on the first surface 2802 aligned with the break-lines 3902 on the second surface 2804, in other embodiments they may not be aligned. While Figure 41 illustrates the bubbles 210 as solid bubbles 210, in other embodiments, the bubbles 210 may be hollow. While Figure 41 illustrates the liquid-impermeable layer 205 as being formed by a single polymer film, in other embodiments the liquid-impermeable layer 205 may be comprise a first sheet of polymeric film and a second sheet of polymeric film, with inner surfaces attached. Although not shown here, some embodiments may further comprise fenestrations, which may be located between bubbles 210. In some embodiments, the fenestrations may be located in the gaps 2808 and/or in the break-lines 3902.

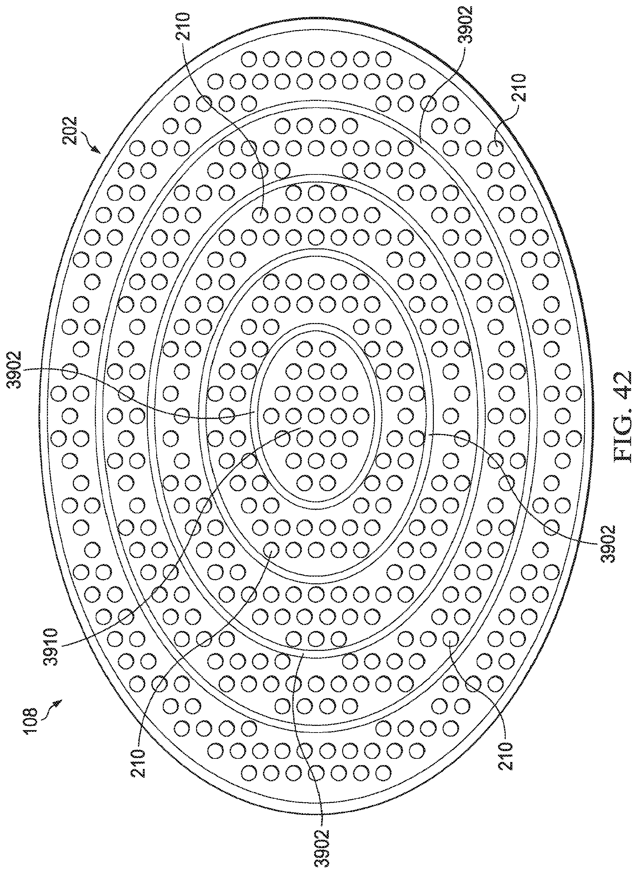

Figure 42 is a schematic, plan view of an illustrative embodiment of still another tissue interface 108 that may be associated with some embodiments of the therapy system 100 of Figure 1, illustrating additional details that may be associated with some embodiments. The bubble manifold 202 of Figure 42 may be similar to that of Figure 39, except with a different pattern for the break-lines 3902. In some embodiments, one or more of the break-lines 3902 may be substantially perpendicular to the fluid flowpath and/or the radius of the liquid-impermeable layer 205. In some embodiments, one or more of the break-lines 3902 may be curved. For example, in Figure 42, all of the break-lines 3902 may be curved. In some embodiments, one or more of the break-lines 3902 may be circular or oval in shape and/or may be disposed around (e.g. encircling) the center 3910 of the liquid-impermeable layer 205. In some embodiments, at least two of the break-lines 3902 may be circular or oval in shape, may be disposed around the center 3910 of the liquid-impermeable layer 205, and/or may be concentrically spaced apart. In some embodiments, adjacent circular or oval break-lines 3902 may be spaced apart approximately 60 mm. In the embodiment shown in Figure 42, all of the break-lines 3902 may be circular or oval in shape, may be disposed around the center 3910 of the liquid-impermeable layer 205, and/or may be concentrically spaced apart. In some embodiments, the break-line pattern shown in Figure 42 may be an oval pattern, and may have an average fluid removal rate of approximately 166.67 mL/min under negative-pressure therapy.

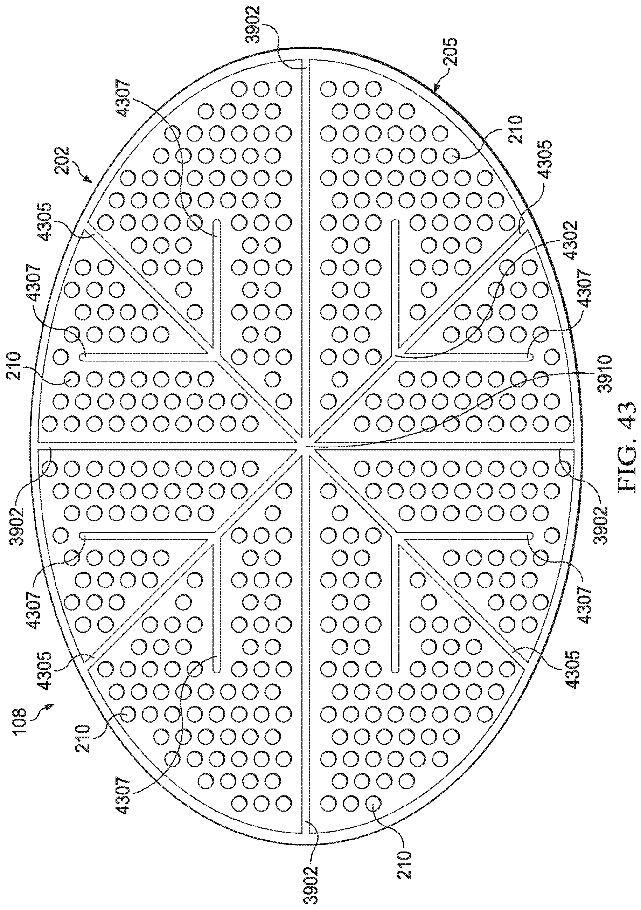

Figure 43 is a schematic, plan view of an illustrative embodiment of still another tissue interface 108 that may be associated with some embodiments of the therapy system 100 of Figure 1, illustrating additional details that may be associated with some embodiments. The bubble manifold 202 of Figure 43 may be similar to that of Figure 39, except with still another different pattern for the break-lines 3902. In some embodiments, at least some of the break-lines 3902 may extend radially. For example, radially-extending break-lines 4305 may be spaced apart approximately 45 degrees and/or may extend from substantially the center 3910 of the liquid-impermeable layer 205 to substantially the perimeter 3912 or periphery of the liquid-impermeable layer 205.

In some embodiments, the pattern of break-lines 3902 may further comprise at least some branch break-lines 4307 each extending off of one of the radially-extending break-lines 4305. For example, one or more branch break-line 4307 may extend outward substantially at an angle less than 90 degrees (e.g. approximately 45 degrees) from at least half of the radially extending break-line 4305. In some embodiments, at least one branch break-line 4307 may extend outward at an angle less than 90 degrees (e.g. approximately 45 degrees) from approximately a midpoint 4302 of four or more radially-extending break-lines 4305. In some embodiments, the break-lines 3902 may further comprise (e.g. in addition to the radially-extending break-lines 4305) two branch break-lines 4307 extending outward from opposite sides at substantially at an angle less than 90 degrees (e.g. approximately 45 degrees) from an approximate midpoint 4302 of four or more radially-extending break-lines 4305. For example, every other (e.g. alternating) radially-extending break-line 4305 may have two branch break-lines 4307 extending outward at approximately 45 degrees from opposite sides of the midpoint 4302 of the corresponding radially-extending break-line 4305. In this context, midpoint may comprise the exact midpoint, or substantially or approximately the midpoint, such as a point in proximity to the exact midpoint of the radially-extending break-line 4305. In some embodiments, the break-line 3902 pattern shown in Figure 43 may be an arrow pattern, and may have an average fluid removal rate of approximately 480.56 mL/min under negative-pressure therapy.

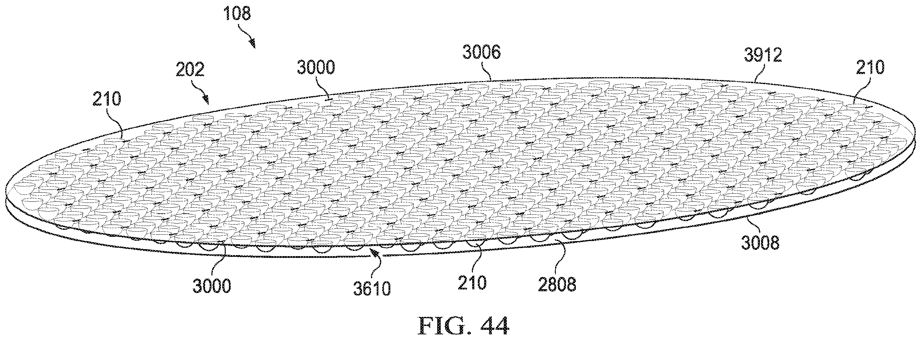

Figure 44 is an isometric view of an illustrative embodiment of yet another tissue interface 108 that may be associated with some embodiments of the therapy system 100 of Figure 1, illustrating additional details that may be associated with some embodiments. Figure 44 illustrates an embodiment having an internal fluid pathway 3610, in which the plurality of bubbles 210 extend from one or both inner surfaces of the two sheets of polymeric film 3006, 3008 to form the internal fluid pathway 3610 located between the inner surfaces of the two sheets of polymeric film 3006, 3008. For example, the tissue interface 108 may comprise: the first sheet 3006 of polymeric film and the second sheet 3008 of polymeric film, with the first plurality of bubbles 210 of the inner surface of the first sheet 3006 of polymeric film contacting the inner surface of the second sheet 3008 of polymeric film, and gaps 2808 between the bubbles 210 forming the internal fluid pathway 3610 between the first sheet 3006 of polymeric film and the second sheet 3008 of polymeric film.

In some embodiments, the bubbles 210 may be configured to resist complete collapse of the internal fluid pathway 3610 during negative-pressure therapy. In some embodiments, at least one of the first sheet 3006 of polymeric film and the second sheet 3008 of polymeric film may be fenestrated (e.g. with fenestrations 3000) to provide fluid communication between the internal fluid pathway 3610 and the tissue site. In some embodiments, the perimeter 3912 or periphery of the tissue interface 108 may not be sealed. For example, the perimeter 3912 of the internal fluid pathway 3610 may be open, without attachment of the perimeter 3912 of the first sheet 3006 to the perimeter 3912 of the second sheet 3008. In some embodiments, there may be no attachment of the first sheet 3006 to the second sheet 3008 within approximately 3 cm of the perimeter 3912.

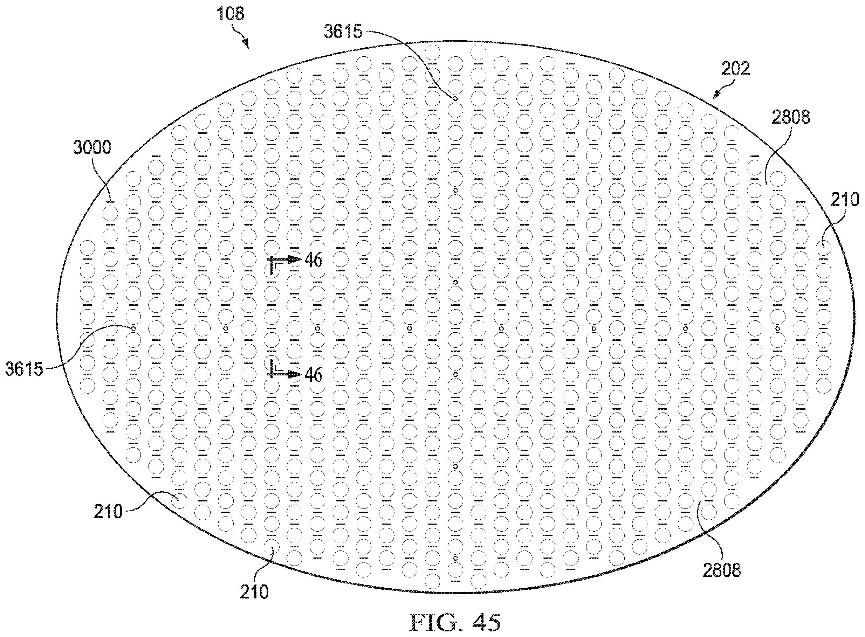

Figure 45 is a schematic, plan view of an illustrative embodiment of the tissue interface 108 of Figure 44, illustrating additional details that may be associated with some embodiments. In some embodiments, the inner surface of the first sheet 3006 of polymeric film and the inner surface of the second sheet 3008 of polymeric film may be attached. In some embodiments, the first sheet 3006 of polymeric film and the second sheet 3008 of polymeric film may be attached in the gaps 2808 between bubbles 210 For example, the first sheet 3006 and the second sheet 3008 may be attached by spot welds 3615 in some of the gaps 2808 between bubbles 210. In some embodiments, the spot welds 3615 may be spaced approximately 2-3 inches apart. In some embodiments, the first sheet 3006 and the second sheet 3008 may be attached along two or more radial lines, for example along two perpendicular radial lines. For example, the spot welds 3615 may be located along two perpendicular radial lines which extend substantially the diameter of the tissue interface 108. In some embodiments, the first sheet 3006 and the second sheet 3008 may be attached by approximately 10-15 spot welds, for example by 12 spot welds 3615. In other embodiments, not shown here, the inner surface of the first sheet 3006 may be attached to the inner surface of the second sheet 3008 by a pattern of break-lines. For example, welds attaching the first sheet 3006 to the second sheet 3008 may form break-lines, similar to those described above. In other embodiments, not shown here, the inner surface of the first sheet 3006 and the second sheet 3008 may be attached at the distal cap ends (e.g. bubble crowns) of the bubbles 210, similar to Figure 38, for example with no attachment in the gaps 2808 between bubbles 210.

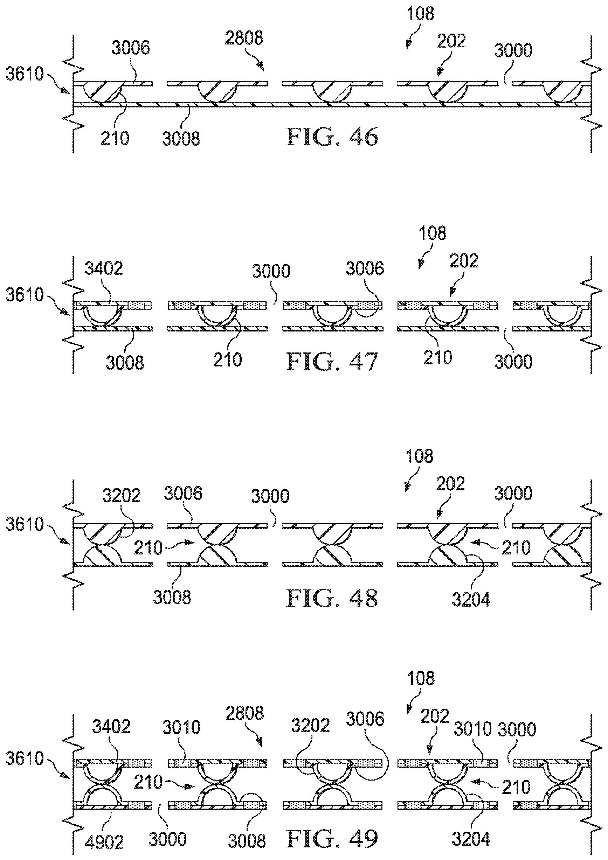

Figure 46 is a section view of an example embodiment cross-section of Figure 45, illustrating additional details that may be associated with some embodiments. Figure 46 illustrates an embodiment in which only the inner surface of the first sheet 3006 has bubbles 210 extending inward toward the second sheet 3008. As shown in Figure 46, the plurality of bubbles 210 may be configured to provide vertical spacing between the stacked first sheet 3006 and second sheet 3008, supporting the inner surface of the second sheet 3008 over the inner surface of the first sheet 3006 to form the internal fluid pathway 3610 between the first sheet 3006 and the second sheet 3008. The internal fluid pathway 3610 may be configured to provide an open fluid pathway in the gaps 2808 between the bubbles 210, which may support the vertical spacing between the first sheet 3006 and the second sheet 3008 under negative-pressure therapy. The bubbles 210 may be configured to resist collapse and/or to maintain an open internal fluid pathway 3610 during negative-pressure therapy. In some embodiments, the internal fluid pathway 3610 may have a height (e.g. vertical spacing) of approximately one bubble, and for example may substantially maintain its height during negative-pressure therapy. In some embodiments, only one sheet may comprise fenestrations 3000, although other embodiments may have fenestrations in both the first sheet 3006 and the second sheet 3008. In some embodiments, the bubbles 210 may be solid, although in other embodiments the bubbles 210 may be hollow.

Figure 47 is a section view of another example embodiment cross-section of Figure 45, illustrating additional details that may be associated with some embodiments. The embodiment of Figure 47 may be similar to Figure 46, and may further comprise hollow bubbles 210 and a third sheet 3402 of polymeric film sealingly attached to the outer surface of the first sheet 3006 of polymeric film (e.g. to seal the bubbles and form closed-cells). In some embodiments, the bubbles 210 extending from the inner surface of the first sheet 3006 may be hollow (e.g. indentations in the first sheet 3006 projecting from the inner surface of the first sheet 3006), with open bubble cavities, and the bubbles 210 may be sealed to form closed-cell bubbles 210 by attachment of the third sheet 3402 to the outer surface of the first sheet 3006. For example, the third sheet 3402 may be welded to the outer surface of the first sheet 3006. In some embodiments, sealing the third sheet 3402 to the outer surface of the first sheet 3006 may seal the bubbles 210 to form closed-cell bubbles 210. For example, the third sheet 3402 may be welded to the first sheet 3006 in the gaps 2808 between bubbles 210. In some embodiments, the fenestrations 3000 may pass through the first sheet 3006 and the third sheet 3402 (e.g. through the welds 3010 attaching the first sheet 3006 and the third sheet 3402), to provide fluid communication between the internal fluid pathway 3610 and the tissue site. Some embodiments may further include fenestrations through the second sheet 3008.

Figure 48 is a section view of an alternate example embodiment cross-section of a tissue interface 108 with bubbles 210 forming an internal fluid pathway 3610, illustrating additional details that may be associated with some embodiments. Some embodiments, as shown in Figure 48, may be similar to Figure 46 and further comprise additional bubbles 210 disposed on the inner surface of the second sheet 3008 of polymeric film. For example, a first set 3202 of bubbles 210 may extend from the inner surface of the first sheet 3006, and a second set 3204 of bubbles 210 may extend from the inner surface of the second sheet 3008. In some embodiments, the plurality of bubbles 210 may comprise the first set 3202 of bubbles 210 and the second set 3204 of bubbles 210. In some embodiments, the first set 3202 of bubbles 210 and the second set 3204 of bubbles 210 may be aligned and stacked (e.g. with the first set 3202 of bubbles 210 contacting the second set 3204 of bubbles 210, typically at their crown ends), which may result in the internal fluid pathway 3610 being two bubbles 210 high. In other embodiments, the bubbles 210 may not be aligned and/or may be offset (e.g. with the first set 3202 of bubbles 210 contacting the second sheet 3008 in the gaps 2808 between the second set 3204 of bubbles 210 and/or the second set 3204 of bubbles 210 contacting the first sheet 3006 in the gaps 2808 between the first set 3202 of bubbles 210), which may result in the internal fluid pathway 3610 being only one bubble high. While the bubbles 210 in Figure 48 are shown as being solid, in other embodiments, the bubbles 210 may be hollow. While fenestrations 3000 are shown in Figure 48 disposed in both the first sheet 3006 and the second sheet 3008, in other embodiments the fenestrations may be located in only one of the sheets.

Figure 49 is a section view of another alternate example embodiment cross-section of a tissue interface 108, illustrating additional details that may be associated with some embodiments. As shown in Figure 49, some tissue interface 108 embodiments may be similar to Figure 48, but configured with hollow bubbles 210. The formation of the hollow bubbles 210 may be similar to those in Figure 47. For example, the first set 3202 of bubbles 210 extending from the inner surface of the first sheet 3006 may be hollow (e.g. indentations in the first sheet 3006 projecting from the inner surface of the first sheet 3006), with open bubble cavities, and the first set 3202 of bubbles 210 may be sealed to form closed-cell bubbles 210 by attachment of the third sheet 3402 to the outer surface of the first sheet 3006. For example, the third sheet 3402 may be welded to the outer surface of the first sheet 3006. In some embodiments, the third sheet 3402 may be welded to the first sheet 3006 in the gaps 2808 between first set 3202 of bubbles 210. In some embodiments, the fenestrations 3000 may pass through the first sheet 3006 and third sheet 3402 (e.g. through the welds 3010 attaching the first sheet 3006 and the third sheet 3402), to provide fluid communication between the internal fluid pathway 3610 and the tissue site.

**In** some embodiments, the second set 3204 of bubbles 210 extending from the inner surface of the second sheet 3008 may be hollow (e.g. indentations in the second sheet 3008 projecting from the inner surface of the second sheet 3008), with open bubble cavities, and the second set 3204 of bubbles 210 may be sealed to form closed-cell bubbles 210 by attachment of a fourth sheet 4902 to the outer surface of the second sheet 3008. For example, the fourth sheet 4902 may be welded to the outer surface of the second sheet 3008. In some embodiments, the fourth sheet 4902 may be welded to the second sheet 3008 in the gaps 2808 between the second set 3204 of bubbles 210. In some embodiments, the fenestrations 3000 may pass through the second and forth sheets (e.g. through the welds 3010 attaching the second sheet 3008 and the fourth sheet 4902), to provide fluid communication between the internal fluid pathway 3610 and the tissue site.

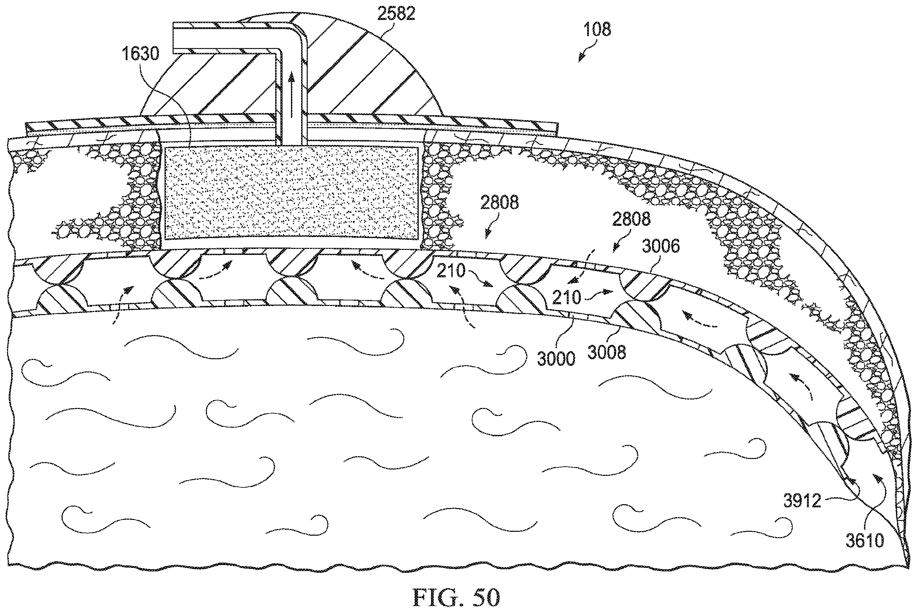

Figure 50 is a schematic diagram, with a portion in cross-section, of an illustrative dressing for treating a tissue site, which may comprise a bubble manifold 202 or tissue interface 108 similar to that of Figure 48 or Figure 49 and may be associated with some embodiments of the therapy system 100 of Figure 1. In some embodiments, a perimeter 3912 or periphery of the internal fluid pathway 3610 may not be sealed. For example, the internal fluid pathway 3610 (e.g. flowpath) may be open at the perimeter 3912. In some embodiments, the inner surfaces of the first sheet 3006 and second sheet 3008 may not be attached for approximately 3 cm around the perimeter 3912. In some embodiments, the perimeter 3912 or periphery of the first sheet 3006 and the second sheet 3008 may be configured for placement within and/or to contact the paracolic gutter. In some embodiments, the inner surfaces of first sheet 3006 and second sheet 3008 may not be attached in a central portion of the tissue interface 108 (e.g. in proximity to the center). For example, the inner surfaces of the first sheet 3006 and the second sheet 3008 may not be attached within approximately 2.5 cm of the center, forming a central portion approximately 5 cm across without welds which may be configured for introduction of negative pressure into the internal fluid pathway 3610. The central portion may comprise fenestrations 3000 or other apertures or openings configured to allow for introduction of negative-pressure into the internal fluid pathway 3610. For example, the foam manifold 1630 may be disposed in contact with the central portion. In some embodiments, the foam manifold 1630 may be disposed between the dressing interface 2582 and the bubble manifold 202, and negative pressure may be applied to the tissue site therethrough. Fenestrations 3000 in one or both of the first sheet 3006 and the second sheet 3008 may allow fluid communication between the internal fluid pathway 3610 and the tissue site (such as the abdominal cavity).

When negative pressure is applied during negative-pressure therapy, fluid (such as exudate) may be drawn into the internal fluid pathway 3610 through the fenestrations 3000 and/or the open perimeter 3912. Under negative pressure, fluid in the internal fluid pathway 3610 may flow (e.g. in the gaps 2808 between bubbles 210) towards the central portion and/or the location of introduction of negative pressure, and may then be drawn out of the internal fluid pathway 3610. In some embodiments, instillation may occur in reverse through the same pathways, for example with instillation fluid exiting the internal fluid pathway 3610 and being distributed outward to the tissue site through the fenestrations 3000 and/or the open perimeter 3912. While Figure 50 illustrates the tissue interface 108 with bubbles 210 on both inner surfaces, other embodiments may have bubbles 210 on only a single inner surface and may function similarly. In some embodiments, a cover (not shown here) may be disposed over the tissue interface 108, for example to seal the tissue site.

In some example method embodiments, forming a tissue interface may comprise: providing a liquid-impermeable layer having a first surface, a second surface, and a plurality of bubbles disposed on at least the first surface; and forming a pattern of break-lines in at least one surface of the liquid-impermeable layer; wherein the break lines divide the plurality of bubbles into a plurality of zones, the bubbles within each zone are approximately uniformly spaced forming a gap between adjacent bubbles, and each beak-line is wider than (e.g. at least twice as wide as) the gap. Some embodiments may further comprise selecting the pattern of break-lines to achieve a desired flow rate. In some embodiments, selecting the pattern may comprise selecting a pattern from the following: a star pattern, an arrow pattern, and an oval pattern. In some embodiments, selecting a pattern may comprise selecting a pattern configured to reduce radial flow rate to less than 647 mL/min or less than 481 mL/min (e.g. approximately 166-481 mL/min).

In some embodiments, providing a liquid-impermeable layer may comprise: providing a first sheet of polymeric film having an inner surface, an outer surface, and the plurality of bubbles on the outer surface; providing a second sheet of polymeric film having an inner surface and an outer surface; and sealingly attaching the inner surfaces together (e.g. to form closed-cell bubbles). In some embodiments, the second sheet of polymer film may be substantially flat (e.g. the outer surface of the second sheet may be substantially flat and/or without bubbles). In some embodiments, the second sheet of polymer film also may have bubbles on its outer surface. In some embodiments, forming a pattern of break-lines in at least one surface of the liquid-impermeable layer may comprise forming a pattern of break-lines on the outer surfaces of the first and second sheets. In some embodiments, forming a pattern of break-lines in at least one surface of the liquid impermeable layer may comprise welding the first and second sheets of polymer film together. In some embodiments, welding the first and second sheets of polymer film together may comprise compressing and heating a portion of the bubbles to form a weld (e.g. using one or more heated platen or roller). In some embodiments, forming a plurality of break-lines may comprise forming bubbles on the at least one surface of the liquid-impermeable layer configured to form the break-line pattern. For example, bubbles may be printed on the surface of the liquid-impermeable layer and spaced to form the break-line pattern. Some method embodiments may comprise forming at least two different tissue interfaces, each having a different break-line pattern and/or flow rate. In some embodiments, two or more such different tissue interfaces may be packaged in a kit. In some embodiments, the kit may further comprise other components of the dressing, such as the cover.

Using a tissue interface for negative-pressure therapy may comprise: determining a recommended flow rate; and selecting the tissue interface from a plurality of available tissue interfaces responsive to the recommended flow rate. In some embodiments, the plurality of available tissue interfaces may have at least two different flow rates. In some embodiments, the plurality of available tissue interfaces may comprise at least two different patterns. For example, the plurality of available patterns may comprise two or more of the following: a tissue interface having no pattern, a tissue interface having an oval or circular pattern, a tissue interface having a star pattern, a tissue interface having an arrow pattern, a tissue interface that is encapsulated, and a tissue interface with internal bubbles. In some embodiments, the recommended flow rate may be greater than 167 and/or less than 647. In some embodiments, the recommended flow rate may be greater than 5 mL/min, greater than 24 mL/min, less than 650 mL/min, less than 481 mL/min, approximately 166-481 mL/min, and/or approximately 208-481 mL/min. In some embodiments, the plurality of available tissue interfaces may not include any pattern perpendicular to the flow direction.

The systems, apparatuses, and methods described herein may provide significant advantages. For example, some embodiments of the tissue interface 108 may include fluid channels comprising bubbles, which may both assist with manifolding fluids and provide stiffness, support, and/or structure to the tissue interface 108. For example, the inclusion of the bubbles as part of the tissue interface may provide good manifolding with a compressible, soft material that can conform to spaces and curves of a tissue site, such as a tissue site within an abdominal cavity. Additionally, the various layers and components of the tissue interface 108 may apply tension and closing force to the abdominal contents, therefore facilitating quicker primary fascia closure of the abdominal cavity. Some embodiments of the tissue interface 108 may reduce manufacturing cost. For example, bubbles in some embodiments of the tissue interface 108, when compressed under negative pressure in an abdominal cavity against a tissue site, may create conduits for transmission of negative pressure and fluid removal, without such conduits having to necessarily be constructed as separate structures into the tissue interface 108 during manufacturing. Additionally, some embodiments of the tissue interface 108 may allow for improved visibility of the underlying tissue site once applied.

In some embodiments, the configuration of the bubbles may improve or increase fluid flow rate and/or better distribute negative pressure during negative-pressure therapy. Some embodiments may be configured with patterns of break-lines, which may allow for control of the fluid flow rate. For example, a tissue interface with a specific break-line pattern may be selected for a specific use, based on the desired flow rate, allowing customization of the flow rate. Some embodiments may be manufactured to minimize larger breaks between bubbles, which may maximize flow rate.

Some embodiments of the tissue interface 108 may be incorporated as part of a dressing that is simple to apply, and can reduce the amount of time needed to size and apply the dressing. Furthermore, some embodiments of the tissue interface 108 disclosed herein may be cut and shaped without exposing foam or other materials that may allow in-growth of tissue and, thus, lead to disruption of the tissue site during dressing removal. Some embodiments of the tissue interface 108 may offer beneficial granulation and a low-trauma and high-seal bond with the tissue site, while substantially eliminating or minimizing incorporation with the tissue site. By incorporating manifolding elements comprising bubbles, such as the closed cells or blisters, possible tissue in-growth of the tissue site into portions of the tissue interface 108 within an abdominal cavity may be significantly reduced or eliminated. Longer application times for the tissue interface 108 and the dressing 102 without adhering to the fascia of abdominal tissue sites may be achieved. Some embodiments of the tissue interface 108 may remain in contact with a tissue site for longer periods of time without undergoing tissue in-growth, and as a result may maintain the ability to be easily removed. Since the tissue interface 108 may also have less mass than previous dressing materials, some embodiments of the tissue interface 108 may be removed through a smaller opening on a patient.

Some embodiments of the tissue interface 108 and dressing 102 may also provide combined temporary abdominal closure with fluid instillation capability. Such embodiments of the tissue interface 108 may therefore provide means for irrigating and cleansing an abdominal cavity while supporting and protecting the abdominal contents, as well as removing contaminated fluid and controlling and/or reducing edema. In some embodiments, the therapy system 100 may provide means for irrigating all areas of an abdominal cavity, including small bowel loops, gutters, retroperitoneal space, portions of the lymphatic system, etc., all while the dressing 102, including the tissue interface 108, is in place, advantageously reducing time required for patients and clinical staff in the operating room. Use of the therapy system 100 may enable exudate and infectious material to be drained from tissue sites, such as those within an abdominal cavity, which can reduce the presence of contaminated abdominal fluids, in order to promote healing. The tissue interface 108 may provide good interaction with tissue at a tissue site, including good manifolding of negative pressure and therapeutic fluids provided in conjunction with instillation therapy. These and other advantages may arise from the disclosure.

If something is described as "exemplary" or an "example", it should be understood that refers to a non-exclusive example. The terms "about" or "approximately" or the like, when used with a number, may mean that specific number, or alternatively, a range in proximity to the specific number as understood by persons of skill in the art field (for example, +/-10%). Use of broader terms such as "comprises", "includes", and "having" should be understood to provide support for narrower terms such as "consisting of", "consisting essentially of", and "comprised substantially of". Use of the term "optionally", "may", "might", "possibly", "could", "can", "would", "should", "preferably", "typically", "often" and the like with respect to any element, component, feature, characteristic, etc. of an embodiment means that the element, component, feature, characteristic, etc. is not required, or alternatively, the element, component, feature, characteristic, etc. is required, both alternatives being within the scope of the embodiment(s). Such element, component, feature, characteristic, etc. may be optionally included in some embodiments, or it may be excluded (e.g. forming alternative embodiments, all of which are included within the scope of disclosure). Section headings used herein are provided for consistency and convenience, and shall not limit or characterize any invention(s) set out in any claims that may issue from this disclosure. If a reference numeral is used to reference a specific example of a more general term, then that reference numeral may also be used to refer to the general term (or vice versa).

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing, the container, or both may be eliminated or separated from other components for manufacture or sale. In other example configurations, the controller may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described in the context of some embodiments may also be omitted, combined, or replaced by alternative features serving the same, equivalent, or similar purpose without departing from the scope of the invention defined by the appended claims. Also, features, elements, and aspects described with respect to a particular embodiment may be combined with features, elements, and aspects described with respect to one or more other embodiments.

## Claims

1. A tissue interface for treating an abdominal tissue site, comprising:
at least one liquid-impermeable layer (205);
a plurality of bubbles (210) disposed on at least a first surface of the liquid-impermeable layer (205); and
a pattern of break-lines (3902, 4305, 4307) configured to separate the plurality of bubbles (210) into a plurality of zones on at least the first surface, wherein each break-line is free of bubbles (210);
wherein at least some of the break-lines (3902, 4305) extend radially and are straight with a width of 3.0 - 24.0mm.

2. The tissue interface of claim 1, wherein the pattern is configured so that no break-lines (3902, 4305, 4307) are substantially perpendicular to a radius of the liquid impermeable layer (205).

3. The tissue interface of claim 1, wherein the liquid-impermeable layer (205) further comprises a second surface opposite the first surface; and the plurality of bubbles (210) extend from both the first surface and the second surface.

4. The tissue interface of claim 3, wherein the pattern of break-lines (3902, 4305, 4307) are formed on both the first surface and the second surface of the liquid-impermeable layer (205).

5. The tissue interface of claim 1, wherein the liquid-impermeable layer (205) further comprises a plurality of fenestrations positioned between the bubbles (210).

6. The tissue interface of claim 1, wherein the pattern of break-lines (3902, 4305, 4307) is configured to define one or more surface flow channels to allow a flow rate of approximately 166-481mL/minute.

7. The tissue interface of claim 1, the liquid-impermeable layer (205) comprises two sheets of polymeric film having inner surfaces attached only in the gaps (2808) between the plurality of bubbles (210), wherein the inner surfaces are attached by welds; and the welds are located entirely within the gaps between the plurality of bubbles.

8. The tissue interface of claim 1, wherein all of the break-lines extend radially, and wherein break lines (3902, 4305) extending from substantially a center of the liquid-impermeable layer (205) to substantially a perimeter of the liquid-impermeable layer (205) are spaced approximately 45 degrees apart.

9. The tissue interface of claim 8, wherein break-lines (3902) additionally extend at least half a distance from substantially the center to substantially the perimeter, and are located substantially half way between the break-lines (3902, 4305) extending from substantially a center of the liquid-impermeable layer (205) to substantially a perimeter of the liquid-impermeable layer (205).

10. The tissue interface of claim 1, wherein the break-lines (3902, 4305, 4307) further comprise at least some break-lines (4307) extending substantially at an angle less than 90 degrees from a midpoint of each radially extending break-line (4305).

11. The tissue interface of claim 1, wherein the bubbles (210) each have a height of approximately 9.0-10.0 mm.

12. The tissue interface of claim 1, wherein the bubbles (210) each have a diameter or width of approximately 6.0-12.0 mm

13. The tissue interface of claim 7, wherein the welds are spot welds spaced apart approximately 5.08-7.62 cm.

14. The tissue interface of claim 1, wherein the pattern of break-lines (3902, 4305, 4307) is configured to define one or more surface flow channels to allow a flow rate greater than 5 mL/minute.

15. The tissue interface of claim 1, wherein the pattern of break-lines (3902, 4305, 4307) is configured to define one or more surface flow channels to allow a flow rate greater than 20 ml/minute.

## Patentansprüche

1. Eine Gewebeschnittstelle zur Behandlung einer abdominalen Gewebestelle, umfassend:
mindestens eine flüssigkeitsundurchlässige Schicht (205);
eine Mehrzahl von Blasen (210), die auf mindestens einer ersten Oberfläche der flüssigkeitsundurchlässigen Schicht (205) angeordnet sind; und
ein Muster von Bruchlinien (3902, 4305, 4307), das konfiguriert ist, um die Mehrzahl von Blasen (210) in eine Mehrzahl von Zonen auf mindestens der ersten Oberfläche zu trennen, wobei jede Bruchlinie frei von Blasen (210) ist;
wobei sich mindestens einige der Bruchlinien (3902, 4305) radial erstrecken und gerade sind mit einer Breite von 3,0 bis 24,0 mm.

2. Die Gewebeschnittstelle nach Anspruch 1, wobei das Muster so konfiguriert ist, dass keine Bruchlinien (3902, 4305, 4307) im Wesentlichen senkrecht zu einem Radius der flüssigkeitsundurchlässigen Schicht (205) stehen.

3. Die Gewebeschnittstelle nach Anspruch 1, wobei die flüssigkeitsundurchlässige Schicht (205) ferner eine zweite Oberfläche gegenüber der ersten Oberfläche umfasst; und die Mehrzahl von Blasen (210) sich von sowohl der ersten Oberfläche als auch der zweiten Oberfläche erstreckt.

4. Die Gewebeschnittstelle nach Anspruch 3, wobei das Muster von Bruchlinien (3902, 4305, 4307) sowohl auf der ersten Oberfläche als auch auf der zweiten Oberfläche der flüssigkeitsundurchlässigen Schicht (205) ausgebildet ist.

5. Die Gewebeschnittstelle nach Anspruch 1, wobei die flüssigkeitsundurchlässige Schicht (205) ferner eine Mehrzahl von Fenestrationen umfasst, die zwischen den Blasen (210) positioniert sind.

6. Die Gewebeschnittstelle nach Anspruch 1, wobei das Muster von Bruchlinien (3902, 4305, 4307) konfiguriert ist, um einen oder mehrere Oberflächenströmungskanäle zu definieren, um eine Strömungsgeschwindigkeit von etwa 166-481 ml/Minute zu ermöglichen.

7. Die Gewebeschnittstelle nach Anspruch 1, wobei die flüssigkeitsundurchlässige Schicht (205) zwei Bögen aus Polymerfolie umfasst, die Innenoberflächen aufweisen, die nur in den Zwischenräumen (2808) zwischen der Mehrzahl von Blasen (210) befestigt sind, wobei die Innenoberflächen durch Schweißnähte befestigt sind; und die Schweißnähte vollständig innerhalb der Zwischenräume zwischen der Mehrzahl von Blasen angeordnet sind.

8. Die Gewebeschnittstelle nach Anspruch 1, wobei sich alle Bruchlinien radial erstrecken, und wobei Bruchlinien (3902, 4305), die sich von im Wesentlichen einem Zentrum der flüssigkeitsundurchlässigen Schicht (205) zu im Wesentlichen einem Umfang der flüssigkeitsundurchlässigen Schicht (205) erstrecken, etwa 45 Grad voneinander beabstandet sind.

9. Die Gewebeschnittstelle nach Anspruch 8, wobei sich Bruchlinien (3902) zusätzlich mindestens über die Hälfte einer Entfernung von im Wesentlichen dem Zentrum zu im Wesentlichen dem Umfang erstrecken und im Wesentlichen auf halbem Weg zwischen den Bruchlinien (3902, 4305) angeordnet sind, die sich von im Wesentlichen einem Zentrum der flüssigkeitsundurchlässigen Schicht (205) zu im Wesentlichen einem Umfang der flüssigkeitsundurchlässigen Schicht (205) erstrecken.

10. Die Gewebeschnittstelle nach Anspruch 1, wobei die Bruchlinien (3902, 4305, 4307) ferner mindestens einige Bruchlinien (4307) umfassen, die sich im Wesentlichen in einem Winkel von weniger als 90 Grad von einem Mittelpunkt jeder sich radial erstreckenden Bruchlinie (4305) erstrecken.

11. Die Gewebeschnittstelle nach Anspruch 1, wobei die Blasen (210) jeweils eine Höhe von etwa 9,0-10,0 mm aufweisen.

12. Die Gewebeschnittstelle nach Anspruch 1, wobei die Blasen (210) jeweils einen Durchmesser oder eine Breite von etwa 6,0-12,0 mm aufweisen.

13. Die Gewebeschnittstelle nach Anspruch 7, wobei die Schweißnähte Punktschweißnähte sind, die etwa 5,08-7,62 cm beabstandet sind.

14. Die Gewebeschnittstelle nach Anspruch 1, wobei das Muster von Bruchlinien (3902, 4305, 4307) konfiguriert ist, um einen oder mehrere Oberflächenströmungskanäle zu definieren, um eine Strömungsgeschwindigkeit größer als 5 ml/Minute zu ermöglichen.

15. Die Gewebeschnittstelle nach Anspruch 1, wobei das Muster von Bruchlinien (3902, 4305, 4307) konfiguriert ist, um einen oder mehrere Oberflächenströmungskanäle zu definieren, um eine Strömungsgeschwindigkeit größer als 20 ml/Minute zu ermöglichen.

## Revendications

1. Interface tissulaire pour le traitement d'un site de tissu abdominal, comprenant :
au moins une couche imperméable aux liquides (205) ;
une pluralité de bulles (210) disposées sur au moins une première surface de la couche imperméable aux liquides (205) ; et
un motif de lignes de rupture (3902, 4305, 4307) conçu pour séparer la pluralité de bulles (210) en une pluralité de zones sur au moins la première surface, dans laquelle chaque ligne de rupture est dépourvue de bulles (210) ;
dans laquelle au moins certaines des lignes de rupture (3902, 4305) s'étendent radialement et sont droites avec une largeur de 3,0 à 24,0 mm.

2. Interface tissulaire selon la revendication 1, dans laquelle le motif est conçu de sorte qu'aucune ligne de rupture (3902, 4305, 4307) ne soit sensiblement perpendiculaire à un rayon de la couche imperméable aux liquides (205).

3. Interface tissulaire selon la revendication 1, dans laquelle la couche imperméable aux liquides (205) comprend en outre une seconde surface opposée à la première surface ; et la pluralité de bulles (210) s'étendent à la fois depuis la première surface et la seconde surface.

4. Interface tissulaire selon la revendication 3, dans laquelle le motif de lignes de rupture (3902, 4305, 4307) est formé à la fois sur la première surface et sur la seconde surface de la couche imperméable aux liquides (205).

5. Interface tissulaire selon la revendication 1, dans laquelle la couche imperméable aux liquides (205) comprend en outre une pluralité de fenestrations positionnées entre les bulles (210).

6. Interface tissulaire selon la revendication 1, dans laquelle le motif de lignes de rupture (3902, 4305, 4307) est conçu pour définir un ou plusieurs canaux d'écoulement de surface pour permettre un débit d'écoulement de surface d'environ 166 à 481 ml/minute.

7. Interface tissulaire selon la revendication 1, la couche imperméable aux liquides (205) comprend deux feuilles de film polymère ayant des surfaces internes fixées uniquement dans les espaces (2808) entre la pluralité de bulles (210), dans laquelle les surfaces internes sont fixées par des soudures ; et les soudures sont situées entièrement à l'intérieur des espaces entre la pluralité de bulles.

8. Interface tissulaire selon la revendication 1, dans laquelle la totalité des lignes de rupture s'étendent radialement, et dans laquelle les lignes de rupture (3902, 4305) s'étendant depuis sensiblement un centre de la couche imperméable aux liquides (205) jusqu'à sensiblement un périmètre de la couche imperméable aux liquides (205) sont espacées d'environ 45 degrés les unes des autres.

9. Interface tissulaire selon la revendication 8, dans laquelle les lignes de rupture (3902) s'étendent en outre sur au moins la moitié d'une distance depuis sensiblement le centre jusqu'à sensiblement le périmètre, et sont situées sensiblement à mi-chemin entre les lignes de rupture (3902, 4305) s'étendant depuis sensiblement un centre de la couche imperméable aux liquides (205) jusqu'à sensiblement un périmètre de la couche imperméable aux liquides (205).

10. Interface tissulaire selon la revendication 1, dans laquelle les lignes de rupture (3902, 4305, 4307) comprennent en outre au moins certaines lignes de rupture (4307) s'étendant sensiblement selon un angle inférieur à 90 degrés depuis un point médian de chaque ligne de rupture s'étendant radialement (4305).

11. Interface tissulaire selon la revendication 1, dans laquelle les bulles (210) ont chacune une hauteur d'environ 9,0 à 10,0 mm.

12. Interface tissulaire selon la revendication 1, dans laquelle les bulles (210) ont chacune un diamètre ou une largeur d'environ 6,0 à 12,0 mm

13. Interface tissulaire selon la revendication 7, dans laquelle les soudures sont des soudures par points espacées d'environ 5,08 à 7,62 cm.

14. Interface tissulaire selon la revendication 1, dans laquelle le motif de lignes de rupture (3902, 4305, 4307) est conçu pour définir un ou plusieurs canaux d'écoulement de surface pour permettre un débit supérieur à 5 ml/minute.

15. Interface tissulaire selon la revendication 1, dans laquelle le motif de lignes de rupture (3902, 4305, 4307) est conçu pour définir un ou plusieurs canaux d'écoulement de surface pour permettre un débit supérieur à 20 ml/minute.
